# FASCICULE DE BREVET EUROPEEN

(11) **EP 1 912 988 B1**
(45) Date de publication et mention de la délivrance du brevet: **16.04.2014**
(21) Numéro de dépôt: 06794254.0
(22) Date de dépôt: 01.08.2006
(51) Int. Cl.: C07D 471/04, A61K 31/437, A61P 35/00, C07D 213/85

(54) **7-AZA-INDAZOLES SUBSTITUES, COMPOSITIONS LES CONTENANT, PROCEDE DE FABRICATION ET UTILISATION**
7-SUBSTITUIERTE AZA-INDAZOLE, DIESE ENTHALTENDE ZUSAMMENSETZUNGEN, HERSTELLUNG UND VERWENDUNG
7-SUBSTITUTED AZA-INDAZOLES, COMPOSITIONS CONTAINING SAME, PRODUCTION METHOD AND USE THEREOF

(30) Priorité: 04.08.2005 FR 0508316
(43) Date de publication de la demande: 23.04.2008
(73) Titulaire: Aventis Pharma S.A., 92160 Antony (FR)
(72) Inventeur: BJERGARDE, Kirsten, Oro Valley, Arizona 85737 (US); NAIR, Anil, Oro Valley, Arizona 85737 (US); PATEK, Marcel, Tucson, Arizona 85704 (US); DODSON, Mark, Oro Valley, Arizona 85737 (US); ACKERMAN-BERRIER, Martha, Tucson, Arizona 85745 (US); SMRCINA, Martin, Tucson, Arizona 85737 (US); LEROY, Vincent, F-94130 Nogent Sur Marne (FR); BACQUE, Eric, F-91190 Gif Sur Yvette (FR); TABART, Michel, F-91290 La Norville (FR); RONAN, Baptiste, F-92140 Clamart (FR); VIVIANI, Fabrice, F-95380 Louvres (FR); SOUAILLE, Catherine, F-94600 Choisy Le Roi (FR)
(74) Mandataire: Blot, Philippe Robert Emile
(86) Numéro de dépôt international: PCT/FR2006/001861
(87) Numéro de publication internationale: WO 2007/017577

(56) Documents cités:
- WO-A-01/19828
- WO-A-03/008405
- WO-A-03/027095
- WO-A-2004/096130
- WO-A-2005/063768
- WO-A-2005/073232
- WO-A-2006/003276
- WO-A-2006/118231
- DD-A1- 154 538
- DE-A1- 2 538 950
- DE-A1- 2 701 610
- GB-A- 1 302 287
- JP-A- 4 117 362
- SENNITSKAYA, L. V. ET AL: "IR-spectroscopic study of the structure of indazoles, pyrazolo[3,4-b]pyridines, and pyrazolo[3,4-b]pyrazine" KHIMIYA GETEROTSIKLICHESKIKH SOEDINENII , (5), 662-7 CODEN: KGSSAQ; ISSN: 0132-6244, 1977, XP001248556
- HOLLAND, GERALD F. ET AL: "Heterocyclic tetrazoles, a new class of lipolysis inhibitors" JOURNAL OF MEDICINAL CHEMISTRY , 10(2), 149-54 CODEN: JMCMAR; ISSN: 0022-2623, 1967, XP002186552
- SARKAR, TARUN K. ET AL: "A Pummerer-based generation and trapping of furo[3,4-c]pyridines: an approach to nitrogen containing heterocyclic analogues of 1-arylnaphthalene lignans" TETRAHEDRON LETTERS , 43(7), 1341-1344 CODEN: TELEAY; ISSN: 0040-4039, 2002, XP004333918
- REIMANN ET AL: "CONFORMATIONALLY RESTRICTED PETHIDINE ANALOGS. PART 6. SYNTHESIS AND PHARMACOLOGICAL EXAMINATION OF TRANS-OCTAHYDROBENZOÄGÜISOQUINOLINE" SCIENTIA PHARMACEUTICA, OESTERREICHISCHE APOTHEKER VERLAGSGESELLSCHAFT,, AT, vol. 64, no. 3-4, 1996, pages 637-646, XP009076539 ISSN: 0036-8709
- SZCZEPANKIEWICZ, BRUCE G. ET AL: "Aminopyridine-Based c-Jun N-Terminal Kinase Inhibitors with Cellular Activity and Minimal Cross-Kinase Activity" JOURNAL OF MEDICINAL CHEMISTRY , 49(12), 3563-3580 CODEN: JMCMAR; ISSN: 0022-2623, 2006, XP002412969

## Description

La présente invention concerne notamment de nouveaux composés chimiques, particulièrement de nouveaux 7-aza-indazoles substitués, des compositions les contenant, et leur utilisation comme médicaments.

Plus particulièrement, l'invention concerne de nouveaux 7-aza-indazoles spécifiques présentant une activité anticancéreuse, via la modulation de l'activité de protéines, en particulier des kinases.

A ce jour, la plupart des composés commerciaux utilisés en chimiothérapie posent des problèmes importants d'effets secondaires et de tolérance par les patients. Ces effets pourraient être limités dans la mesure où les médicaments utilisés agissent sélectivement sur les cellules cancéreuses, à l'exclusion des cellules saines. Une des solutions pour limiter les effets indésirables d'une chimiothérapie peut donc consister en l'utilisation de médicaments agissant sur des voies métaboliques ou des éléments constitutifs de ces voies, exprimés majoritairement dans les cellules cancéreuses, et qui ne seraient pas ou peu exprimés dans les cellules saines.

Les protéines kinases sont une famille d'enzymes qui catalysent la phosphorylation de groupes hydroxyles de résidus spécifiques de protéines tels que des résidus tyrosine, sérine ou thréonine. De telles phosphorylations peuvent largement modifier la fonction des protéines ; ainsi, les protéines kinases jouent un rôle important dans la régulation d'une grande variété de processus cellulaires, incluant notamment le métabolisme, la prolifération cellulaire, la différentiation cellulaire, la migration cellulaire ou la survie cellulaire. Parmi les différentes fonctions cellulaires dans lesquelles l'activité d'une protéine kinase est impliquée, certains processus représentent des cibles attractives pour traiter les maladies cancéreuses ainsi que d'autres maladies.

Ainsi, un des objets de la présente invention est de proposer des compositions ayant une activité anticancéreuse, en agissant en particulier vis-à-vis de kinases. Parmi les kinases pour lesquelles une modulation de l'activité est recherchée, FAK, KDR et Tie2 sont préférées.

Ces produits répondent à la formule (I) suivante : 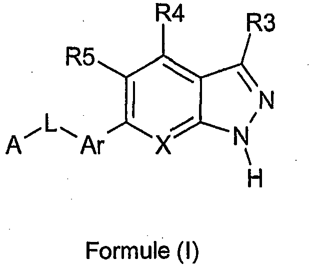 dans laquelle :
1) A et Ar sont indépendamment sélectionnés dans le groupe constitué par : aryle, hétéroaryle, hétérocyclyle, aryle substitué, hétéroaryle substitué, hétérocyclyle substitué, cycloalkyle, et cycloalkyle substitué ;
2) L est sélectionné dans le groupe constitué par : NH, NH-SO₂, SO₂NH, NH-CH₂, CH₂-NH, NH-CO, CO-NH, CH₂-CO-NH, NH-CO-CH₂, NH-CH₂-CO, CO-CH₂-NH, NH-CO-NH, NH-CS-NH, NH-CO-O, O-CO-NH, CH₂-NH-CO-NH, NH-CO-NH-CH₂, et NH-CO-CH₂-CO-NH;
3) X est N ou NO ;
4) R3 est sélectionné dans le groupe constitué par H et NHMR"3, dans lequel M est sélectionné dans le groupe constitué par : liaison, CO, CO-NH, CS, CS-NH, SO₂; et dans lequel R"3 est sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, et hétérocyclyle substitué ;
5) R4 est sélectionné dans le groupe constitué par : H, halogène, alkyle, alkyle substitué, OR"4 , N(R"5)(R"6), CON(R"5)(R"6), , dans lequel R"4 est choisi parmi H, phényle, phényle substitué, alkyle, alkyle substitué, et dans lequel R"5 et R"6 sont indépendamment sélectionnés dans le groupe constitué par H, (C1-C6)alkyle, (C1-C6)alkyle substitué, -(C1-C6)alkyle-hétérocyclyle, -(C1-C6)alkyle-hétérocyclyle substitué, -(C1-C6)alkyle-hétéroaryle, -(C1-C6)alkyle-hétéroaryle substitué, cycloalkyle, cycloalkyle substitué, hétérocyclyle, hétérocyclyle substitué, aryle, aryle substitué, hétéroaryle, hétéroaryle substitué ou bien R"5 et R"6 sont liés entre eux pour former un cycle saturé de 4 à 8 chaînons contenant de 1 à 3 hétéroatomes choisis parmi O, S et N, éventuellement substitué;
6) R5 est sélectionné dans le groupe constitué par : H, halogène, R'2, CN, O(R'2), OC(O)(R'2), OC(O)N(R'2)(R'3), OS(O₂)(R'2), N(R'2)(R'3), N=C(R'2)(R'3), N(R'2)C(O)(R'3), N(R'2)C(O)O(R'3), N(R'4)C(O)N(R'2)(R'3), N(R'4)C(S)N(R'2)(R'3), N(R'2)S(O₂)(R'3), C(O)(R'2), C(O)O(R'2), C(O)N(R'2)(R'3), C(=N(R'3))(R'2), C(=N(OR'3))(R'2), S(R'2), S(O)(R'2), S(O₂)(R'2), S(O₂)O(R'2), S(O₂)N(R'2)(R'3) ; dans lequel chaque R'2, R'3, R'4 est indépendamment sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, hétérocyclyle substitué; et R'2 et R'3 peuvent être liés entre eux pour former un cycle contenant de 1 à 3 hétéroatomes choisis parmi O, S et N ; À la condition que lorsque X est N, R3 est NH₂, Ar et A sont phényle non substitué, L est NHCO lié en para de Ar, et R5 est H, alors R4 n'est pas choisi parmi : phényle, *o*-chlorophényle, cinnamyle, α-furfuryle, *o*-hydroxyphényle, *p-*hydroxy-*m*-méthoxyphényle, *p*-méthylthiophényle, *p*-méthoxyphényle, *o-*nitrophényle, *m*-phénoxyphényle, et à la condition que lorsque X est N, R5 est H, R4 est H, et Ar-L-A est un groupe 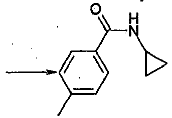 alors R3 n'est pas choisi parmi : amino, acétylamino, [(4-fluorophényl)carbonyl]amino, (2-méthylpropanoyl)amino, -(cyclopentylcarbonyl)amino, propanoylamino, [(4-méthylphényl)carbonyl]amino, {[4-(methyloxy)phenyl]carbônyl}amino, (2-thiénylcarbonyl)amino, (méthylsulfonyl)amino, -[(4-fluorophényl)sulfonyl]amino, (éthylsulfonyl)amino, (propylsulfonyl)amino, (3-thiénylsulfonyl)amino, [(3,5-diméthyl-4-isoxazolyl)sulfonyl]amino, (2-thiénylsulfonyl)amino, (1-méthyléthyl)amino.

Des produits de formule (I) préférés répondent à la définition suivante : 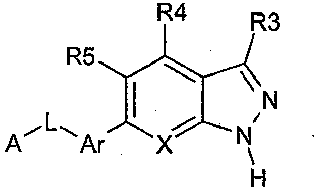 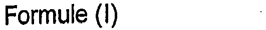 dans laquelle :
1) A et Ar sont indépendamment sélectionnés dans le groupe constitué par : aryle, hétéroaryle, hétérocyclyle, cycloalkyle, aryle substitué, hétéroaryle substitué, hétérocyclyle substitué, et cycloalkyle substitué ;
2) L est sélectionné dans le groupe constitué par : NH, NH-SO₂, SO₂NH, NH-CH₂, CH₂-NH, CH₂-CO-NH, NH-CO-CH₂, NH-CH₂-CO, CO-CH₂-NH, NH-CO-NH, NH-CS-NH, NH-CO-O, O-CO-NH, CH₂-NH-CO₋NH, NH-CO-NH-CH₂, et NH-CO-CH₂-CO-NH;
3) X est N ;
4) R3 est sélectionné parmi H , NH2 et NHCOR"3 et R"3 sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, et hétérocyclyle substitué ;
5) R4 est sélectionné dans le groupe constitué par : H, halogène, alkyle, alkyle substitué, CON(R"5)(R"6) dans lequel R"5 et R"6 sont indépendamment sélectionnés dans le groupe constitué par H, (C1-C6)alkyle, (C1-C6)alkyle substitué, -(C1-C6)alkyle-hétérocyclyle, -(C1-C6)alkyle-hétérocyclyle substitué, - (C1-C6)alkyle-hétéroaryle, -(C1-C6)alkyle-hétéroaryle substitué, cycloalkyle, cycloalkyle substitué, hétérocyclyle, hétérocyclyle substitué, aryle, aryle substitué, hétéroaryle, hétéroaryle substitué ou bien R"5 et R"6 sont liés entre eux pour former un cycle saturé de 4 à 8 chaînons contenant de 1 à 3 hétéroatomes choisis parmi O, S et N, éventuellement substitué ;
6) R5 est H.

Dans les produits de formule (I), Ar est choisi parmi un thiazolyle, thiényle, furyle, pyrrolyle, oxazolyle, isoxazolyle, isothiazolyle, thiadiazolyle, pyrazolyle, imidazolyle, indolyle, indazolyle, benzimidazolyle, benzoxazolyle, et benzothiazolyle ; éventuellement substitué, ou bien Ar est un thiazolyle, ou bien Ar- L-A est : 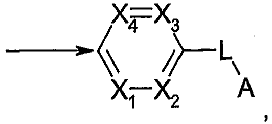
dans lequel chaque X1, X2, X3 et X4 est indépendamment choisi parmi N et C-R'5, dans lequel R'5 a la même définition que R5.

Des substituants L-A préférés sont avantageusement choisis parmi NH-CO-NH-A et NH-SO₂-A. Une combinaison L-A particulièrement efficace est obtenue lorsque L-A est NHCONH-A.

Des produits conformes à l'invention ont de préférence un substituant A qui est sélectionné dans le groupe constitué par phényle, pyridyle, pyrimidyle, thiényle, furyle, pyrrolyle, oxazolyle, thiazolyle, isoxazolyle, isothiazolyle, pyrazolyle, imidazolyle, indolyle, indazolyle, benzimidazolyle, benzoxazolyle, et benzothiazolyle ; éventuellement substitué.

De manière plus préférée, A est choisi parmi phényle, pyrazolyle et isoxazolyle ; éventuellement substitué.

Le substituant A est très avantageusement substitué par un premier substituant sélectionné dans le groupe constitué par halogène, alkyle, alkylène, alkynyle, aryle, hétéroaryle, O-alkyle, O-Aryle, O-hétéroaryle, S-alkyle, S-Aryle, S-hétéroaryle, chacun étant éventuellement substitué par un ou plusieurs substituant choisi parmi (C1-C3)alkyle, halogène, O-(C1-C3)alkyle.

Le substituant A est préférentiellement substitué par un deuxième substituant sélectionné dans le groupe constitué par F, Cl, Br, I, OH, SH, SO₃M, COOM, CN, NO₂, CON(R8)(R9), N(R8)CO(R9), (C1-C3)alkyle-OH, (C1-C3)alkyle-N(R8)(R9), (C1-C3)alkyle-(R10), (C1-C3)alkyle-COOH, N(R8)(R9); dans lequel R8 et R9 sont indépendamment choisis parmi H, (C1-C3)alkyle, (C1-C3)alkyle halogéné, (C1-C3)alkyleOH, (C1-C3)alkyle-O(C1-C3)alkyle, (C1-C3)alkyleNH₂, (C1-C3)alkyleN(R8)(R9), (C1-C3)alkyleCOOM, (C1-C3)alkyleSO₃M ; dans lequel lorsque R8 et R9 sont simultanément différents de H, ils peuvent être liés pour former un cycle de 5 à 7 chaînons contenant de 1 à 3 hétéroatomes ; dans lequel M est H ou un cation de métal alcalin choisi parmi Li, Na et K ; et dans lequel R10 est H ou un hétérocycle non aromatique éventuellement substitué, comprenant 2 à 7 atomes de carbone, et 1 à 3 hétéroatomes choisis parmi N, O et S.

Des substituants A particulièrement préférés sont choisis parmi phényle, pyrazolyle et isoxazolyle ; lesdits substituants A pouvant être substitués par halogène, (C1-C4)alkyle, (C1-C3)alkyle halogéné, O-(C1-C4)alkyle, S-(C1-C4)alkyle, O-(C1-C4)alkyle halogéné, et S-(C1-C4)alkyle halogéné. Lorsque A est disubstitué, les deux substituants de A peuvent former un cycle de 5 à 7 chaînons contenant de 0 à 3 hétéroatomes choisis parmi O, N et S.
Un substituant R4 est avantageusement sélectionné dans le groupe constitué par H et CON(R"5)(R"6), avec R"5 et R"6 tels que définis précédemment.

Un produit conforme à l'invention pourra se présenter sous forme :
a) non chirale, ou
b) racémique, ou
c) enrichie en un stéréoisomère, ou
d) enrichie en un énantiomère ;
et pourra être éventuellement salifié.
Un produit conforme à l'invention pourra être utilisé pour la fabrication d'un médicament utile pour traiter un état pathologique, en particulier un cancer. Un objet de la présente invention est un médicament caractérisé en ce qu'il comprend un produit de formule (I) ou un sel d'addition de ce composé à un acide pharmaceutiquement acceptable, ou encore un hydrate ou un solvate du produit de formule (I).

La présente invention concerne aussi les compositions thérapeutiques comprenant un produit selon l'invention, en combinaison avec un excipient pharmaceutiquement acceptable selon le mode d'administration choisi. La composition pharmaceutique peut se présenter sous forme solide, liquide ou de liposomes.

Parmi les compositions solides on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants.

Les formes liquides seront, de préférence, injectables et de ce fait auront une formulation acceptable pour une telle utilisation.

Des voies d'administration par injection acceptables incluent les voies intraveineuse, intra-péritonéale, intramusculaire, et sous cutanée, la voie intraveineuse étant habituellement préférée.

La dose administrée des composés de l'invention sera adaptée par le praticien en fonction de la voie d'administration au patient et de l'état de ce dernier.

Grâce à leur faible toxicité et leurs propriétés pharmacologiques et biologiques, les composés de la présente invention trouvent leur application dans le traitement de tout carcinome ayant un degré de vascularisation important ou induisant des métastases ou enfin dans des pathologies de type lymphomes et leucémies.

Ces composés représentent une thérapie de choix soit seul soit en association avec une chimiothérapie ou radiothérapie adaptée et/ou en association avec d'autres composés possédant des activités anti-angiogènes comme les inhibiteurs des VEGFs ou des FGFs. Ainsi les produits de formule générale (I) sont notamment utiles pour le traitement ou la prévention d'un état pathologique caractérisé en ce que le produit est administré seul ou en association avec d'autres principes actifs notamment des anti-cancéreux tels que des produits cytotoxiques, cytostatiques, anti-angiogéniques, ou anti-métastasiques.

Les composés de la présente invention peuvent donc être administrés seuls ou en mélange avec d'autres anticancéreux. Parmi les associations possibles on peut citer:
- les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine
- les dérivés du platine comme notamment le cisplatine, le carboplatine ou l'oxaliplatine
- les agents antibiotiques comme notamment la bléomycine, la mitomycine, la dactinomycine
- les agents antimicrotubules comme notamment la vinblastine, la vincristine, la vindésine, la vinorelbine, les taxoïdes (paclitaxel et docétaxel)
- les anthracyclines comme notamment la doxorubicine, la daunorubicine, l'idarubicine, l'épirubicine, la mitoxantrone, la losoxantrone
- les inhibiteurs de topoisomérases des groupes I est II telles que l'étoposide, le teniposide, l'amsacrine, l'irinotecan, le topotecan et le tomudex
- les fluoropyrimidines telles que la 5-fluorouracile, l'UFT, la floxuridine
- les analogues de cytidine telles que la 5-azacytidine, la cytarabine, la gemcitabine, la 6-mercaptomurine, la 6-thioguanine
- les analogues d'adénosine tels que la pentostatine, la cytarabine ou le phosphate de fludarabine
- le méthotrexate et l'acide folinique
- les enzymes et composés divers tels que la L-asparaginase, l'hydroxyurée, l'acide trans-rétinoique, la suramine, la dexrazoxane, l'amifostine, l'herceptine ainsi que les hormones oestrogéniques, androgéniques
- les agents antivasculaires tels que les dérivés de la combretastatine, par exemple la CA4P, des chalcones ou de la colchicine, par exemple le ZD6126, et leurs prodrogues
- les agents anti-angiogéniques tels que le bevacizumab, le sorafenib ou le sunitinib malate
- les agents thérapeutiques inhibiteurs d'autres tyrosine kinases tels que l'imatinib, le gefitinib et l'erlotinib.

Lorsque les composés de la présente invention sont associés à un autre traitement ou à un traitement par des radiations, ces traitements peuvent alors être administrés simultanément, séparément, séquentiellement. Le traitement sera adapté par le praticien en fonction du malade à traiter. ,

Les produits de l'invention sont utiles comme agents inhibiteurs d'une réaction catalysée par une ou plusieurs kinases. FAK, KDR et Tie2 sont des kinases pour lesquelles les produits de l'invention seront particulièrement utiles en tant qu'inhibiteurs.

Les raisons pour lesquelles ces kinases sont choisies sont données ci-après :

### FAK

FAK est une tyrosine kinase cytoplasmique jouant un rôle important dans la transduction du signal transmis par les intégrines, famille de récepteurs hétérodimériques de l'adhésion cellulaire. FAK et les intégrines sont colocalisés dans des structures périmembranaires appelées plaques d'adhérence. Il a été montré dans de nombreux types cellulaires que l'activation de FAK ainsi que sa phosphorylation sur des résidus tyrosine et en particulier son autophosphorylation sur la tyrosine 397 étaient dépendantes de la liaison des intégrines à leurs ligands extracellulaires et donc induites lors de l'adhésion cellulaire [Kornberg L, et al. J. Biol. Chem. 267(33): 23439-442. (1992)]. L'autophosphorylation sur la tyrosine 397 de FAK représente un site de liaison pour une autre tyrosine kinase, Src, via son domaine SH2 [Schaller et al. Mol. Cell. Biol. 14:1680-1688. 1994; Xing et al. Mol. Cell. Biol. 5 :413-421. 1994]. Src peut alors phosphoryler FAK sur la tyrosine 925, recrutant ainsi la protéine adaptatrice Grb2 et induisant dans certaines cellules l'activation de la voie ras et MAP Kinase impliquée dans le contrôle de la prolifération cellulaire [Schlaepfer et al. Nature; 372:786-791. 1994; Schlaepfer et al. Prog. Biophy. Mol. Biol. 71:435-478. 1999; Schlaepfer and Hunter, J. Biol. Chem. 272:13189-13195. 1997]. L'activation de FAK peut aussi induire la voie de signalisation jun NH2-terminal kinase (JNK) et résulter dans la progression des cellules vers la phase G1 du cycle cellulaire [Oktay et al., J. Cell. Biol.145:1461-1469. 1999]. Phosphatidylinositol-3-OH kinase (PI3-kinase) se lie aussi à FAK sur la tyrosine 397 et cette interaction pourrait être nécessaire à l'activation de PI3-kinase [Chen and Guan, Proc. Nat. Acad. Sci. USA. 91: 10148-10152. 1994; Ling et al. J. Cell. Biochem. 73:533-544. 1999]. Le complexe FAK/Src phosphoryle différents substrats comme la paxilline et p130CAS dans les fibroblastes [Vuori et al. Mol. Cell. Biol. 16: 2606-2613. 1996].

Les résultats de nombreuses études soutiennent l'hypothèse que les inhibiteurs de FAK pourraient être utiles dans le traitement du cancer. Des études ont suggéré que FAK pourrait jouer un rôle important dans la prolifération et/ou la survie cellulaires *in vitro.* Par exemple, dans les cellules CHO, certains auteurs ont démontré que la surexpression de p125FAK conduit à une accélération de la transition G1 à S, suggérant que p125FAK favorise la prolifération cellulaire [Zhao J.-H et al. J. Cell Biol. 143:1997-2008. 1998]. D'autres auteurs ont montré que des cellules tumorales traitées avec des oligonucleotides anti-sens de FAK perdent leur adhésion et entrent en apoptose (Xu et al, Cell Growth Differ. 4:413-418. 1996). Il a également été démontré que FAK promeut la migration des cellules *in vitro.* Ainsi, des fibroblastes déficients pour l'expression de FAK (souris « knockout » pour FAK) présentent une morphologie arrondie, des déficiences de migration cellulaire en réponse à des signaux chimiotactiques et ces défauts sont supprimés par une réexpression de FAK [DJ. Sieg et al., J. Cell Science. 112:2677-91. 1999]. La surexpression du domaine C-terminal de FAK (FRNK) bloque l'étirement des cellules adhérentes et réduit la migration cellulaire *in vitro* [Richardson A. and Parsons J.T. Nature. 380:538-540. 1996]. La surexpression de FAK dans des cellules CHO, COS ou dans des cellules d'astrocytome humain favorise la migration des cellules. L'implication de FAK dans la promotion de la prolifération et de la migration des cellules dans de nombreux types cellulaires *in vitro,* suggère le rôle potentiel de FAK dans les processus néoplasiques. Une étude récente a effectivement démontré l'augmentation de la prolifération des cellules tumorales *in vivo* après induction de l'expression de FAK dans des cellules d'astrocytome humain [Cary L.A. et al. J. Cell Sci. 109:1787-94. 1996; Wang D et al. J. Cell Sci. 113:4221-4230. 2000]. De plus, des études immunohistochimiques de biopsies humaines ont démontré que FAK était surexprimé dans les cancers de la prostate, du sein, de la thyroïde, du colon, du mélanome, du cerveau et du poumon, le niveau d'expression de FAK étant directement corrélé aux tumeurs présentant le phénotype le plus agressif [Weiner TM, et al. Lancet. 342(8878):1024-1025. 1993 ; Owens et al. Cancer Research. 55:2752-2755. 1995; Maung K. et al. Oncogene. 18:6824-6828. 1999; Wang D et al. J. Cell Sci. 113:4221-4230. 2000].

### KDR

KDR (Kinase insert Domain Receptor) aussi appelée VEGF-R2 (Vascular Endothelial Growth Factor Receptor 2), est exprimé uniquement dans les cellules endothéliales. Ce récepteur se fixe au facteur de croissance angiogénique VEGF, et sert ainsi de médiateur à un signal transductionnel via l'activation de son domaine kinase intracellulaire. L'inhibition directe de l'activité kinase de VEGF-R2 permet de réduire le phénomène d'angiogénèse en présence de VEGF exogène (Vascular Endothelial Growth Factor : facteur de croissance vasculaire endothélial) (Strawn et al., Cancer Research, 1996, vol. 56, p.3540-3545). Ce processus a été démontré notamment à l'aide de mutants VEGF-R2 (Millauer et al., Cancer Research, 1996, vol. 56, p.1615-1620). Le récepteur VEGF-R2 semble n'avoir aucune autre fonction chez l'adulte que celle liée à l'activité angiogénique du VEGF. Par conséquent, un inhibiteur sélectif de l'activité kinase du VEGF-R2 ne devrait démontrer que peu de toxicité.

En plus de ce rôle central dans le processus dynamique angiogénique, des résultats récents suggèrent que l'expression de VEGF contribue à la survie des cellules tumorales après des chimio- et radio-thérapies, soulignant la synergie potentielle d'inhibiteurs de KDR avec d'autres agents (Lee et al. Cancer Research, 2000, vol. 60, p.5565-5570).

### Tie2

Tie-2 (TEK) est un membre d'une famille de récepteurs à tyrosine kinase, spécifique des cellules endothéliales. Tie2 est le premier récepteur à activité tyrosine kinase dont on connaît à la fois l'agoniste (angiopoïetine 1 ou Ang1) qui stimule l'autophosphorylation du récepteur et la signalisation cellulaire [S. Davis et al (1996) Cell 87, 1161-1169] et l'antagoniste (angiopoïetine 2 ou Ang2) [P.C. Maisonpierre et al. (1997) Science 277, 55-60*].* L'angiopoïetine 1 peut synergiser avec le VEGF dans les derniers stades de la néo-angiogénèse [AsaharaT. Circ. Res.(1998) 233-240]. Les expériences de knock-out et les manipulations transgéniques de l'expression de Tie2 ou de Ang1 conduisent à des animaux qui présentent des défauts de vascularisation [D.J. Dumont et al (1994) Genes Dev. 8, 1897-1909 *et* C. Suri (1996) Cell 87, 1171-1180*],* La liaison d'Ang1 à son récepteur conduit à l'autophosphorylation du domaine kinase de Tie2 qui est essentielle pour la néovascularisation ainsi que pour le recrutement et l'interaction des vaisseaux avec les péricytes et les cellules musculaires lisses ; ces phénomènes contribuent à la maturation et la stabilité des vaisseaux nouvellement formés [P.C. Maisonpierre et al (1997) Science 277, 55-60*].* Lin et al (1997) J. Clin. Invest. 100, 8: 2072-2078 *et* Lin P. (1998) PNAS 95, 8829-8834*,* ont montré une inhibition de la croissance et de la vascularisation tumorale, ainsi qu'une diminution des métastases de poumon, lors d'infections adénovirales ou d'injections du domaine extracellulaire de Tie-2 (Tek) dans des modèles de xénographes de tumeur du sein et de mélanome.

Pour les raisons qui suivent, les inhibiteurs de Tie2 peuvent être utilisés dans les situations où une néovascularisation ou angiogenèse se fait de façon inappropriée c'est-à-dire dans les cancers en général mais aussi dans des cancers particuliers tels que le sarcome de Kaposi ou l'hémoangiome infantile, l'arthrite rhumatoïde, l'osthéoarthrite et/ou ses douleurs associées, les maladies inflammatoires de l'intestin telle que la recto-colite hémorragique ou la maladie de Crohn's, les pathologies de l'oeil telle que la dégénérescence maculaire liée à l'âge, les rétinopathies diabétiques, l'inflammation chronique, le psoriasis.
L'angiogenèse est un processus de génération de nouveaux vaisseaux capillaires à partir des vaisseaux pré-existants. L'angiogenèse tumorale (formation de néovaisseaux sanguins), indispensable à la croissance tumorale, est également un des facteurs essentiels de la dissémination métastatique (Oncogene. 2003 May 19;22(20):3172-9 ; Nat Med. 1995 Jan;1(1):27-31.).
Cette néo-vascularisation est due à la migration, puis à la prolifération et à la différenciation des cellules endothéliales sous l'influence de facteurs angiogéniques sécrétés par les cellules cancéreuses et les cellules du stroma (Recent Prog Horm Res. 2000;55:15-35; 35-6).
Le système angiopoïétine 1 / récepteur Tie2 joue un rôle prépondérant dans la maturation des vaisseaux en permettant le recrutement de cellules péri-endothéliales pour stabiliser le vaisseau (Cell. 1996 Dec 27;87(7):1161-9, Recent Prog Horm Res. 2004;59:51-71). Ainsi il a été montré que l'administration de la forme recombinante soluble du domaine extracellulaire du récepteur Tie-2 (exTek) inhibe inhibe l'angiogenèse tumorale dans des modèles de tumeurs murines ainsi que le développement métastatique (Proc Natl Acad Sci USA. 1998 Jul 21;95(15):8829-34 ; Cancer immunol Immunother. 2004 Jul;53(7):600-8). Dans des cellules endothéliales en culture, la stimulation de Tie-2 active la voie de la PI3 kinase, de p42/p44 voies impliquées dans la prolifération et la migration cellulaire; de la synthèse de PAF (Cell Signal. 2006 Apr 14; ahead of print) voie impliquée dans l'activité pro-inflammatoire. La stimulation de Tie2 stimule la voie de l'Akt et inhibe l'apoptose (Exp Cell Res. 2004 Aug 1;298(1):167-77) une voie de transduction connue pour son importance dans la survie cellulaire.
L'ajout de Extek (récepteur soluble de Tie2) inhibe la formation de pseudo tubules des cellules endothéliales sur Matrigel (Cancer immunol Immunother. 2004 Jul; 53(7): 600-8. Ces études indiquent que le système Tie-2/Angiopoiétine est nécessaire lors des premiers stades de la formation de bourgeons vasculaires dans les tissus adultes et qu'une fonction du récepteur Tie-2 est d'augmenter la survie des cellules endothéliales au cours de la formation des vaisseaux sanguins. De plus, l'angiopoietine-1 stimule la prolifération des cellules endothéliales lymphatiques ainsi que la lymphangiogenèse (développement des néo-vaisseaux lymphatiques) voie d'accès privilégiés pour le développement métastatique (Blood. 2005 Jun 15; 105(12): 4649-56)

Les processus d'angiogenèse joue ainsi un rôle prépondérant dans la progression de nombreuses tumeurs solides. De plus, il a été montré que la probabilité d'apparition de métastases augmente très fortement avec l'augmentation de la vascularisation de la tumeur primaire (Br J Cancer. 2002 May 20 ; 86(10): 1566-77. Le rôle potentiel d'agents pro-angiogéniques dans les leucémies et lymphomes a été également et plus récemment documenté. En effet de manière générale il a été rapporté que des clones cellulaires dans ces pathologies peuvent être soit détruits naturellement par le système immunitaire soit basculer dans un phénotype angiogénique qui favorise leur survie puis leur prolifération. Ce changement de phénotype est induit par une sur expression de facteurs angiogéniques notamment par les macrophages et/ou une mobilisation de ces facteurs à partir de la matrice extracellulaire (Thomas DA, Giles FJ, Cortes J, Albitar M, Kantarjian HM. , Acta Haematoi, (2001), vol 207, pp106-190.
Il existe une corrélation entre le processus d'angiogenèse de la moelle osseuse et les "extramedullar disease" dans les CML (chronic myelomonocytic leukemia). Différentes études démontrent que l'inhibition de l'angiogenèse, pourrait représenter une thérapeutique de choix dans cette pathologie (Leuk Res. 2006 Jan; 30(1): 54-9 ; Histol Histopathol. 2004 oct. ;19(4): 1245-60. De plus, il est fortement suggéré que l'activation du système Tie2/angiopoietine soit impliquée dans le développement de l'angiogenèse de la moelle osseuse chez les patients atteints de myélome multiple (Blood. 2003 Jul 15; 102(2): 638-45.

L'arthrite rhumatoïde (RA) est une maladie chronique avec une étiologie inconnue. Alors qu'elle affecte de nombreux organes, la forme la plus sévère de RA est une inflammation synoviale des articulations progressive aboutissant à la destruction. L'angiogenèse semble affecter de manière importante la progression de cette pathologie. Ainsi, il a été montré que l'activation de Tie2 régule l'angiogenèse dans les tissus synoviaux favorisant le développement de l'arthrite rhumatoïde (Arthritis Rheum. 2003 Sep; 48(9): 2461-71)
Il a été montré également une sur expression de L'angiopoientin 1 et de Tie2 dans les tissus synoviaux de patients atteints d'ostheoarthrite corrélée à une néovascularisation active (Shahrara S et al Arthritis Res. 2002;4(3)). Ainsi, il a été montré qu'en bloquant l'activation de Tie2 par l'utilisation d'un adénovirus produisant exTEK (récepteur Tie2 soluble) on obtient une inhibition de l'angiogenèse, du développement de l'arthrose et une protection de la dégradation osseuse dans un modèle de souris où l'arthrose est induite au collagène (Arthritis Rheum. 2005 May; 52(5):1346-8).
Les IBD (inflammatory bowel disease) comprennent deux formes de maladies inflammatoires chroniques de l'intestin : les UC ( ulcerative colitis) et la maladie de Crohn's (CD). Les IBD sont caractérisées par une dysfonction immunitaire se traduisant par une production inappropriée de cytokines inflammatoires induisant l'établissement d'un système micro-vasculaire local. Cette angiogenèse d'origine inflammatoire a pour conséquence une ischémie intestinale induite par vasoconstriction (Inflamm Bowel Dis. 2006 Jun;12(6):515-23).
Les pathologies de l'oeil en relation avec des phénomènes de néo vascularisation comme la dégenerescence maculaire liée à l'age sont responsables d'une large majorité des cas de cécité dans les pays développés. Les signaux moléculaires qui contrôlent les phénomènes de néo vascularisation dans l'oeil tels que les VEGFs ou les angiopoiétines sont des cibles de choix dans ces pathologies (Campochiaro PA. Expert Opin Biol Ther. 2004 Sep;4(9)). Ainsi, il a été montré qu'en bloquant l'activation de Tie2 par l'utilisation d'un adénovirus produisant exTEK (récepteur Tie2 soluble) inhibait la néovascularisation rétinienne et choroïdienne qui est la cause la plus fréquente de la perte de vision (Hum Gene Ther. 2001 Jul 1; 12(10):1311-21)

### Définitions

Le terme « halogène » fait référence à un élément choisi parmi F, Cl, Br, et I.

Le terme « alkyle » fait référence à un substituant hydrocarboné saturé, linéaire ou ramifié, ayant de 1 à 12 atomes de carbone. Les substituants méthyle, éthyle, propyle, 1-méthyléthyl, butyle, 1-méthylpropyl, 2-méthylpropyle, 1,1-diméthyléthyle, pentyle, 1-méthylbutyle, 2-méthylbutyle, 3-méthylbutyle, 1,1-diméthylpropyle, 1,2-diméthylpropyle, 2,2-diméthyl-propyle, 1-éthylpropyle, hexyle, 1-méthylpentyle, 2-méthylpentyle, 1-éthyl-butyle, 2-éthylbutyle, 3,3-diméthyibutyle, heptyle, 1-éthylpentyle, octyle, nonyle, décyle, undécyle, et dodécyle sont des exemples de substituant alkyle.

Le terme « alkylène » fait référence à un substituant hydrocarboné linéaire ou ramifié ayant une ou plusieurs insaturations, ayant de 2 à 12 atomes de carbone. Les substituants éthylènyle, 1-méthyléthylènyle, prop-1-ènyle, prop-2-ènyle, Z-1-méthylprop-1-ènyle, E-1-méthylprop-1-ènyle, Z-1,2-diméthyl-prop-1-ènyle, E-1,2-diméthylprop-1-ènyle, but-1,3-diényle, 1-méthylidènyl-prop-2-ènyle, Z-2-méthylbut-1,3-diényle, E-2-méthylbut-1,3-diényle, 2-méthyl-1-méthylidènylprop-2-ènyle, undéc-1-ènyle et undéc-10-ènyle sont des exemples de substituant alkylène.

Le terme « alkynyle » fait référence à un substituant hydrocarboné linéaire ou ramifié ayant au moins deux insaturations portées par une paire d'atomes de carbone vicinaux, ayant de 2 à 12 atomes de carbone. Les substituants éthynyle; prop-1-ynyle; prop-2-ynyle; et but-1-ynyle sont des exemples de substituant alkynyle.

Le terme « aryle » fait référence à un substituant aromatique mono- ou polycyclique ayant de 6 à 14 atomes de carbone. Les substituants phényle, napht-1-yle ; napht-2-yle ; anthracen-9-yl ; 1,2,3,4-tétrahydronapht-5-yle ; et 1,2,3,4-tétrahydronapht-6-yle sont des exemples de substituant aryle.

Le terme « hétéroaryle » fait référence à un substituant hétéroaromatique mono- ou polycyclique ayant de 1 à 13 atomes de carbone et de 1 à 4 hétéroatomes. Les substituants pyrrol-1-yle ; pyrrol2-yle ; pyrrol3-yle ; furyle ; thienyle ; imidazolyle ; oxazolyle ; thiazolyle ; isoxazolyle ; isothiazolyle ; 1,2,4-triazolyle ; oxadiazolyle ; thiadiazolyle ; tétrazolyle ; pyridyle ; pyrimidyle ; pyrazinyle ; 1,3,5-triazinyle ; indolyle ; benzo[b]furyle ; benzo[b]thiényle ; indazolyle ; benzimidazolyle ; azaindolyle ; quinoléyle ; isoquinoléyle ; carbazolyle ; et acridyle sont des exemples de substituant hétéroaryle.

Le terme « hétéroatome » fait référence ici à un atome au moins divalent, différent du carbone. N; O; S; et Se sont des exemples d'hétéroatome.

Le terme « cycloalkyle » fait référence à un substituant hydrocarboné cyclique saturé ou partiellement insaturé ayant de 3 à 12 atomes de carbone. Lés substituants cyclopropyle; cyclobutyle; cyclopentyle; cyclopentènyle; cyclopentadiényle; cyclohexyle; cyclohexènyle; cycloheptyle; bicyclo[2.2.1]heptyle ; cyclooctyle; bicyclo[2.2.2]octyle ; adamantyle ; et perhydronapthyle sont des exemples de substituant cycloalkyle.

Le terme « hétérocyclyle » fait référence à un substituant hydrocarboné cyclique saturé ou partiellement insaturé ayant de 1 à 13 atomes de carbone et de 1 à 4 hétéroatomes. De préférence, le substituant hydrocarboné cyclique saturé ou partiellement insaturé sera monocyclique et comportera 4 ou 5 atomes de carbone et 1 à 3 hétéroatomes.

Le terme « substitué » fait référence à un ou plusieurs substituants différents de H, par exemple halogène; alkyle; aryle; hétéroaryle, cycloalkyle ; hétérocyclyle ; alkylène; alkynyle; OH ; O-alkyle; alkyle-OH ; O-alkylène ; O-aryle; O-hétéroaryle; NH₂ ; NH-alkyle; NH-aryle; NH-hétéroaryle; N-alkyle-alkyle' ; SH ; S-alkyle; S-aryle; S(O_{z})H ; S(O₂)-alkyle; S(O₂)-aryle; SO₃H ; SO₃-alkyle; SO₃-aryle; CHO ; C(O)-alkyle; C(O)-aryle ; C(O)OH C(O)O-alkyle; C(O)O-aryle ; OC(O)-alkyle; OC(O)-aryle ; C(O)NH₂; C(O)NH-alkyle; C(O)NH-aryle ; NHCHO ; NHC(O)-alkyle; NHC(O)-aryle ; NH-cycloalkyle ; NH-hétérocyclyle.

La présente invention a encore pour objet les procédés de préparation des produits de formule (1).
Les produits selon l'invention peuvent être préparés à partir de méthodes conventionnelles de chimie organique.
Le schéma 1 ci-dessous est illustratif de la méthode utilisée pour la préparation des exemples 1 et 3. A ce titre, elle ne saurait constituer une limitation de la portée de l'invention, en ce qui concerne les méthodes de préparation des composés revendiqués.

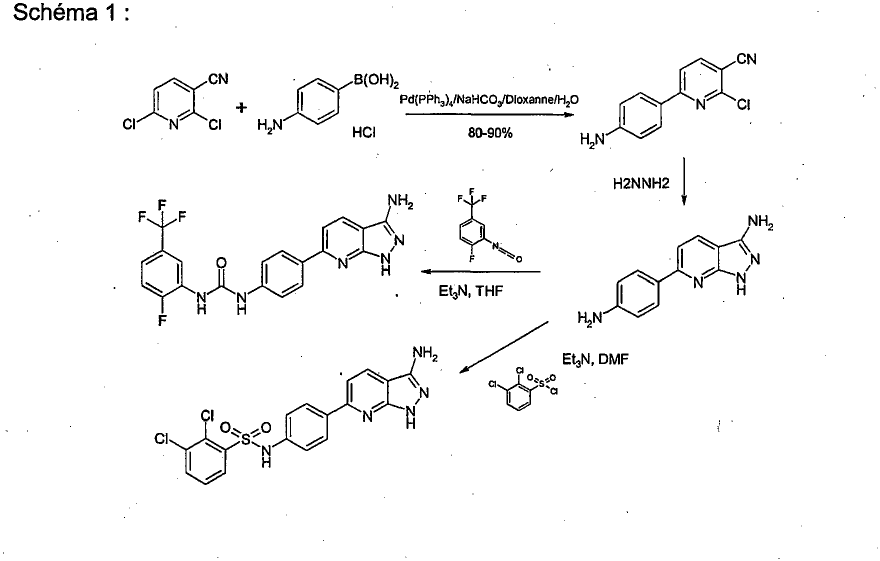

Le schéma 2 ci-dessous est illustratif d'une méthode alternative utilisée pour la préparation de l'exemple 1. A ce titre, elle ne saurait constituer une limitation de la portée de l'invention, en ce qui concerne les méthodes de préparation des composés revendiqués.

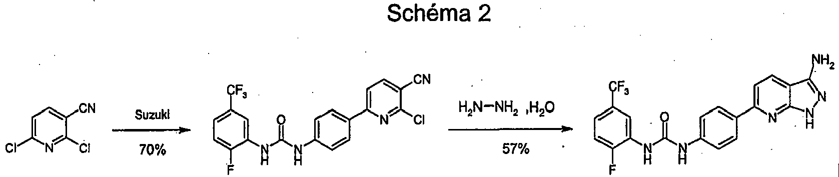

Le schéma 3 ci-dessous est illustratif d'une méthode utilisée pour la préparation des exemples 6 à 8, 20 à 21, 33 à 35, et 38 à 39. A ce titre, elle ne saurait constituer une limitation de la portée de l'invention, en ce qui concerne les méthodes de préparation des composés revendiqués.

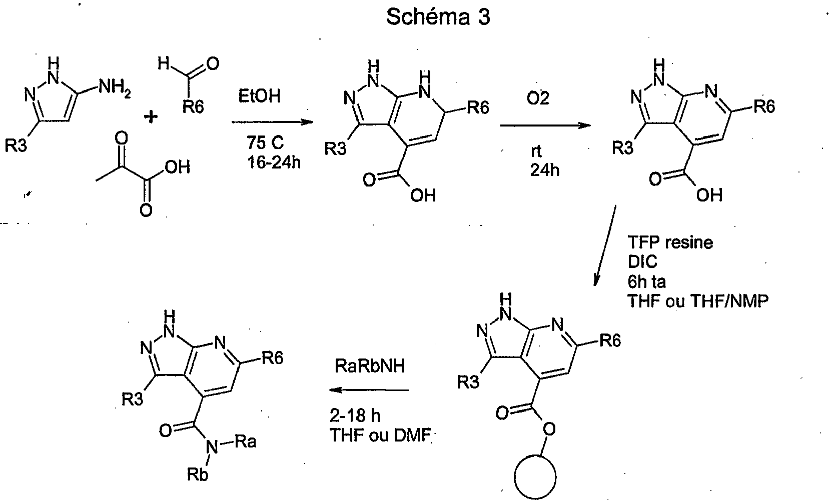

Le schéma 4 ci-dessous est illustratif d'une méthode utilisée pour la préparation des exemples 4, 9 à 19, 22 à 32, 36 à 37, et 44 à 62. A ce titre, elle ne saurait constituer une limitation de la portée de l'invention, en ce qui concerne les méthodes de préparation des composés revendiqués.

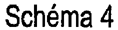

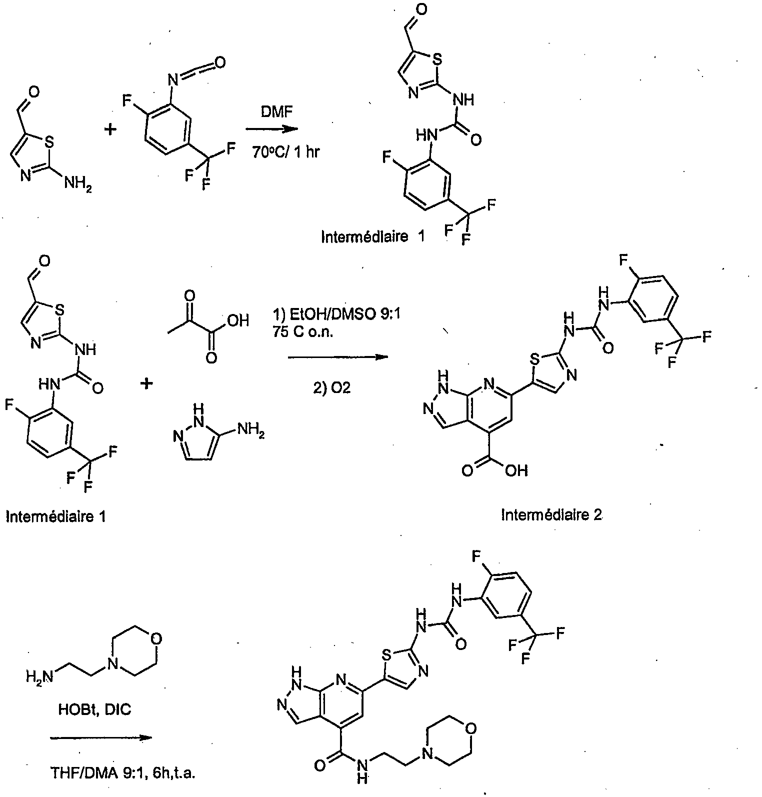

Le schéma 5 ci-dessous est illustratif d'une méthode utilisée pour la préparation des exemples 5, et 40 à 43. A ce titre, elle ne saurait constituer une limitation de la portée de l'invention, en ce qui concerne les méthodes de préparation des composés revendiqués. '

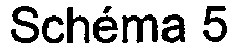

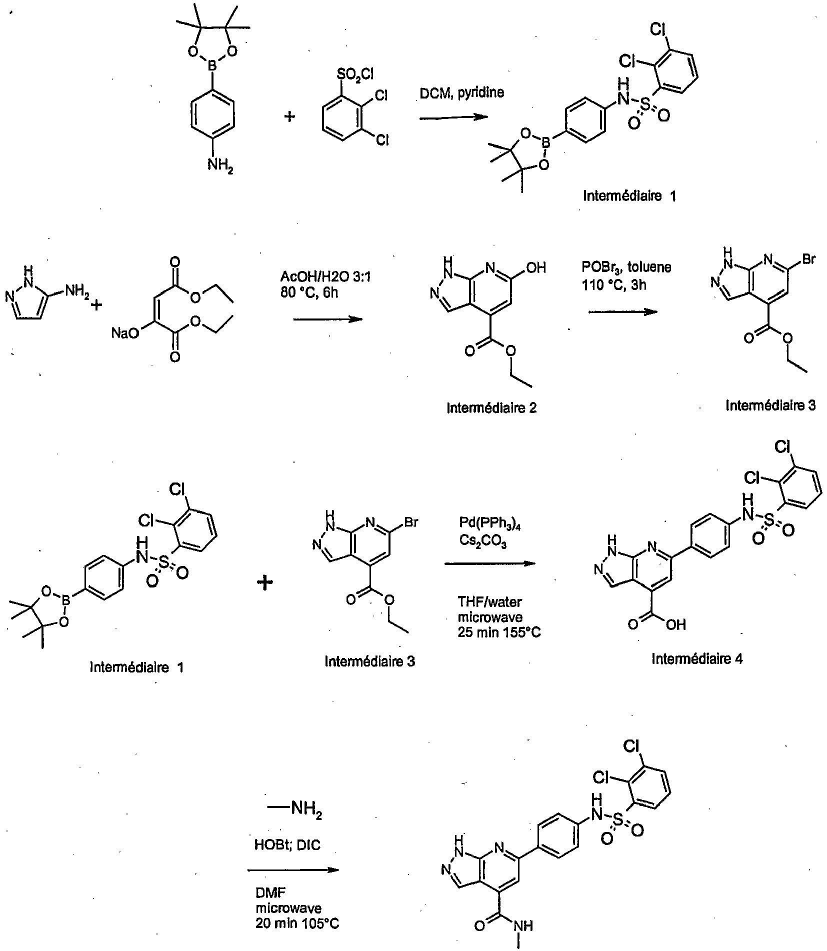

Il est entendu pour l'homme du métier que, pour la mise en oeuvre des procédés selon l'invention décrits précédemment, il peut être nécessaire d'introduire des groupements protecteurs des fonctions amino, carboxyle et alcool afin d'éviter des réactions secondaires. Ces groupes sont ceux qui permettent d'être éliminés sans toucher au reste de la molécule. Comme exemples de groupes protecteurs de la fonction amino, on peut citer le carbamate de *tert*-butyle qui peut être régénéré au moyen d'acide trifluoroacétique ou d'iodotriméthylsilane, l'acétyle qui peut être régénéré en milieu acide (acide chlorhydrique par exemple). Comme groupes protecteurs de la fonction carboxyle, on peut citer les esters (méthoxyméthylester, benzylester par exemple). Comme groupes protecteurs de la fonction alcool, on peut citer les esters (benzoylester par exemple) qui peuvent être régénérés en milieu acide ou par hydrogénation catalyfiique. D'autres groupes protecteurs utilisables sont décrits par T. W. GREENE et coll. dans Protective Groups in Organic Synthesis, third edition, 1999, Wiley-Interscience.

Il est fait mention ici des produits de formule générale (II) suivante : 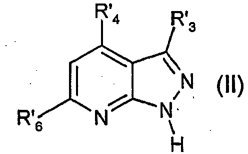
dans laquelle R'₄ représente R4 ou H, -COOH ou -COO-(C1-C6)alkyle, R'₃ représente H, -NH₂ ou -NHCO-thiényle, et R'₆ représente un atome d'halogène, un groupe -Ar-NH₂ où Ar est tel que défini précédemment, ou un groupe Ar-L-A où Ar, L et A sont tels que définis précédemment, et des produits de formule générale (III) suivante : 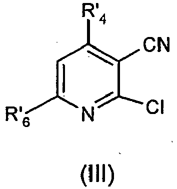
dans laquelle R'₄ représente R4 ou H, -COOH ou -COO-(C1-C6)alkyle, et R'₆ représente un atome d'halogène, un groupe -Ar-NH₂ où Ar est tel que défini précédemment, ou un groupe Ar-L-A où Ar, L et A sont tels que définis précédemment: Ces produits sont notamment utiles comme intermédiaires de synthèse dans les procédés de préparation des produits de formule générale (I).

Les composés de formule (I) sont isolés et peuvent être purifiés par les méthodes connues habituelles, par exemple par cristallisation, chromatographie ou extraction.

Les énantiomères, diastéréoisomères des composés de formule (I) font également partie de l'invention.

Les composés de formule (I) comportant un reste basique peuvent être éventuellement transformés en sels d'addition avec un acide minéral ou organique, par action d'un tel acide au sein d'un solvant, par exemple organique tel un alcool, une cétone, un éther ou un solvant chloré.

Les composés de formule (I) comportant un reste acide peuvent être éventuellement transformés en sels métalliques ou en sels d'addition avec des bases azotées selon les méthodes connues en soi. Ces sels peuvent être obtenus par action d'une base métallique (alcaline ou alcalinoterreuse par exemple), de l'ammoniac, d'une amine ou d'un sel d'amine sur un composé de formule (I), dans un solvant. Le sel formé est séparé par les méthodes habituelles.

Ces sels font également partie de l'invention.

Lorsqu'un produit selon l'invention présente au moins une fonction basique libre, des sels pharmaceutiquement acceptables peuvent être préparés par réaction entre ledit produit et un acide minéral ou organique. Des sels pharmaceutiquement acceptables incluent les chlorures, nitrates, sulfates, hydrogénosulfates, pyrosulfates, bisulfates, sulfites, bisulfites, phosphates, monohydrogénophosphates, dihydrogénophosphates, métaphosphates, pyrophosphates, acétates, propionates, acrylates, 4-hydroxybutyrates, caprylates, caproates, décanoates, oxalates, malonates, succinates, glutarates, adipates, pimélates, maléates, fumarates, citrates, tartrates, lactates, phénylacétates, mandélates, sébacates, subérates, benzoates, phtalates, méthanesulfonates, p-toluène sulfonate, propanesulfonates, xylènesulfonates, salicylates, cinnamates, glutamates, aspartates, glucuronates, galacturonates.

Lorsqu'un produit selon l'invention présente au moins une fonction acide libre, des sels pharmaceutiquement acceptables peuvent être préparés par réaction entre ledit produit et une base minérale ou organique. Des bases pharmaceutiquement acceptables incluent des hydroxydes de cations de métaux alcalins ou alcalino-terreux tels que Li, Na, K, Mg, Ca, des composés aminés basiques tels qu'ammoniac, arginine, histidine, pipéridine, morpholine, pipérazine, triéthylamine.

L'invention est également décrite par les exemples suivants, donnés à titre d'illustration de l'invention.

Les analyses LC/MS ont été réalisées sur un appareil Micromass modèle LCT relié à un appareil HP 1100. L'abondance des produits a été mesurée à l'aide d'un détecteur à barrette de diodes HP G1315A sur une gamme d'onde de 200-600 nm et un détecteur à dispersion de lumière Sedex 65. L'acquisition des spectres de masses Mass spectra a été réalisée sur une gamme de 180 à 800. Les données ont été analysées en utilisant le logiciel Micromass MassLynx. La séparation a été effectuée sur une colonne Hypersil BDS C18, 3 µm (50 x 4.6 mm), en éluant par un gradient linéaire de 5 à 90% d'acétonitrile contenant 0,05% (v/v) d'acide trifluoroacétique (TFA) dans l'eau contenant 0,05% (v/v) TFA en 3,5 mn à un débit de 1 mL/mn. Le temps total d'analyse, incluant la période de rééquilibration de la colonne, est de 7 mn.

Les spectres MS ont été réalisés en électrospray (ES⁺) sur un appareil Platform. Il (Micromass). Les principaux ions observés sont décrits.

Les points de fusion ont été mesurés en capillaire, sur un appareil Mettler FP62, gamme 30°C à 300°C, montée de 2°C par minute.

### Purification par LC/MS:

Les produits peuvent être purifiés par LC/MS en utilisant un système Waters FractionsLynx composé d'une pompe à gradient Waters modèle 600, d'une pompe de régénération Waters modèle 515, d'une pompe de dilution Waters Reagent Manager, d'un auto-injecteur Waters modèle 2700, de deux vannes Rheodyne modèle LabPro, d'un détecteur à barrette de diodes Waters modèle 996, d'un spectromètre de masse Waters modèle ZMD et d'un collecteur de fractions Gilson modèle 204. Le système était controlé par le logiciel Waters FractionLynx. La séparation a été effectuée alternativement sur deux colonnes Waters Symmetry (C₁₈, 5µM, 19x50 mm, référence catalogue 186000210), une colonne étant en cours de régénération par un mélange eau/acétonitrile 95/5 (v/v) contenant 0,07% (v/v) d'acide trifluoroacétique, pendant que l'autre colonne était en cours de séparation. L'élution des colonnes a été effectuée en utilisant un gradient linéaire de 5 à 95% d'acétonitrile contenant 0,07% (v/v) d'acide trifluoroacétique dans l'eau contenant 0,07% (v/v) d'acide trifluoroacétique, à un débit de 10 mL/mn. A la sortie de la colonne de séparation, un millième de l'effluent est séparé par un LC Packing Accurate, dilué à l'alcool méthylique à un débit de 0,5 mL/mn et envoyé vers les détecteurs, à raison de 75% vers le détecteur à barrette de diodes, et les 25% restants vers le spectromètre de masse. Le reste de l'effluent (999/1000) est envoyé vers le collecteur de fractions où le flux est éliminé tant que la masse du produit attendu n'est pas détectée par le logiciel FractionLynx. Les formules moléculaires des produits attendus sont fournies au logiciel FractionLynx qui déclenche la collecte du produit quand le signal de masse détecté correspond à l'ion [M+H]⁺ et/ou au [M+Naj⁺, Dans certains cas, dépendant des résultats de LC/MS analytique, quand un ion intense correspondant à [M+2H]⁺⁺ a été détecté, la valeur correspondant à la moitié de la masse moléculaire calculée (MW/2) est aussi fournie au logiciel FractionLynx. Dans ces conditions, la collecte est aussi déclenchée quand le signal de masse de l'ion [M+2H]⁺⁺ et/ou [M+Na+H]⁺⁺ sont détectés. Les produits ont été collectés en tube de verre tarés. Après collecte, les solvants ont été évaporés, dans un évaporateur centrifuge Savant AES 2000 ou Genevac HT8 et les masses de produits ont été déterminées par pesée des tubes après évaporation des solvants.

### Exemple 1

### 1-[4-(3-Amino-1H-pyrazolo[3,4-b]pyridin-6-yl)-phényl]-3-(2-fluoro-5-trifluorométhyl-phényl)-urée

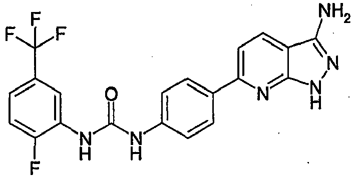

100 mg (0.44 mmol) de 6-(4-Amino-phényl)-1H-pyrazoio[3,4-b]pyridin-3-ylamine sont partiellement mis en solution dans 2.5 mL de THF anhydre. Le mélange est alors dégazé pendant 15 minutes avec de l'argon. 61.55 µL (44.93 mg, 0.44 mmol, 1 éq.) de triéthylamine sont ensuite ajoutés. La solution est encore une fois dégazée 15 minutes. Finalement, 64.22 µL (91 mg, 0.44 mmol, 1 éq.) de 2-Fluoro-5-(trifluorométhyl)phényl-isocyanate sont ajoutés goutte à goutte. Le mélange est agité 2 heures à température ambiante sous atmosphère inerte. Après réaction, le mélange est filtré. Le filtrat est concentré. Un solide beige est isolé. Ce produit brut est ensuite purifié sur colonne de silice (13 g) C18 phase inverse avec un gradient d'éluant de 5 à 95% d'acétonitrile dans l'eau. Les fractions contenant le produit attendu sont lyophilisées. Un solide blanc est isolé. (10 mg).

MS (ES) MH⁺m/z=431.

RMN ¹H RMN (DMSO-d6) δ 5,52 (s large, 2H); 7,39 (m, 1H); 7,49 (m partiellement masqué, 1 H) ; 7,52 (d, J = 8,5 Hz, 1H) ; 7,62 (d large, J = 8,5 Hz, 2H) ; 8,07 (d large, J = 8,5 Hz, 2H) ; 8,13 (d, J = 8,5 Hz, 1H) ; 8,60 (d large, J = 7,5 Hz, 1 H) ; 9,21 (m large, 1 H) ; 9,63 (m large, 1H) ; 11,9 (m étalé, 1 H). 6-(4-Amino-phényl)-1H-pyrazolo[3,4-b]pyridin-3-ylamine :

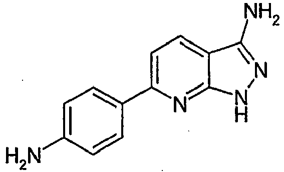

Dans un réacteur pour micro-ondes de taille adaptée, 0.97 mmol (222 mg) de 6-(4-Amino-phényl)-2-chloro-nicotinonitrile est mis en suspension dans 3.3 mL de 1-Propanol. 0.28 mL (0.290 g, 5.80 mmol, 6 éq.) d'hydrate d'hydrazine est ajouté. La suspension est chauffée 45 minutes à 130°C au four à micro-ondes. Le mélange est filtré et le solide est séché pour obtenir un solide vert (215 mg)
MS (IE) M⁺m/z=225.

¹H RMN (DMSO-d6) δ 5.41 (si, 4H), 6.63 (d, J = 8 Hz, 2H), 7.37 (d, J = 8 Hz, 1H), 7.82 (d, J = 8 Hz, 2H), 8.02 (d, J = 2 Hz, 1 H).

### 6-(4-Amino-phényl)-2-chloro-nicôtinonitrile :

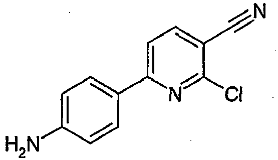

2.89 mmol (500 mg) de 2,6-dichloronicotinonitrile et 3.18 mmol (551 mg, 1.1 éq.) d'acide 4-aminophényl boronique sont mis en solution sous atmosphère inerte dans 33.3 mL de dioxanne. 680 mg (8.09 mmol, 2.8 éq.) de bicarbonate de sodium puis 8.3 mL d'eau sont ensuite ajoutés. Le mélange est agité 5 minutes sous atmosphère inerte puis 334 mg (0.29 mmol, 0.1 eq.) de tétrakis(triphénylphosphine)palladium est ajouté. Le mélange réactionnel est porté au reflux (100°c) pendant 2 heures puis est refroidi à température ambiante. Le milieu réactionnel est filtré puis extrait quatre fois avec de l'acétate d'éthyle. Les phases organiques sont réunies, lavées deux fois avec une solution aqueuse saturée en chlorure de sodium, séchées avec du sulfate de magnésium et concentrées. Un solide jaune est alors obtenu. Ce brut est purifié sur une colonne de 90 g de silice, avec un gradient d'éluant de 20% à 50% d'acétate d'éthyle dans l'heptane. On obtient le produit attendu sous la forme d'un solide jaune (531 mg).
MS (ES) MH⁺m/z=230.
¹H RMN (DMSO-d6) δ 5.94 (sl, 2H), 6.65 (d, j = 8 hz, 2H), 7.89 (d, j = 8 hz, 2H), 7.93 (d, j = 8 hz, 1 H), 8.27 (d, j = 8 hz, 1 H).

Voie alternative pour la préparation de la 1-[4-(3-amino-1H-pyrazolo[3,4-b]pyridin-6-yl)-phényl]-3-(2-fluoro-5-trifluorométhyl-phényl)-urée (schéma 2) :

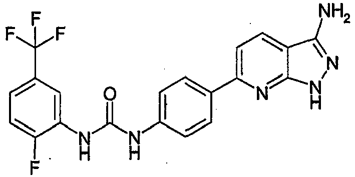

0.46 mmol (200 mg) de 1-[4-(6-Chloro-5-cyano-pyridin-2-yl)-phényl]-3-(2-fluoro-5-trifluorométhyl-phényl)-urée est mis en suspension dans 3.2 mL de 1-propanol. 0.13 mL (0.14 g, 2.76 mmol, 6 éq.) d'hydrate d'hydrazine est ajouté. La suspension est chauffée à 80°C pendant 10h. Le mélange est filtré et le solide est séché pour obtenir 140 mg du produit attendu.

### 1-[4-(6-Chloro-5-cyano-pyridin-2-yl)-phényl]-3-(2-fluoro-5-trifluorométhyl-phényl)-urée :

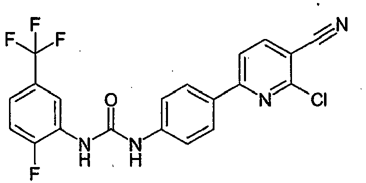

0.578 mmol (100 mg) de 2,6-dichloronicotinonitrile et 0.64 mmol (270 m, 1.1 éq.) de 1-(2-fluoro-5-trifluorométhyl-phényl)-3-[4-(4,4,5,5-tétraméthyl-[1,3,2]dioxaborolan-2-yl)-phényl]-urée sont mis en solution sous atmosphère inerte dans 6.6 mL de dioxane. 136 mg (1.62 mmol, 2.8 éq.) de bicarbonate de sodium puis 1.6 mL d'eau sont ensuite ajoutés. Le mélange est agité 5 minutes sous atmosphère inerte puis 33.4 mg (0.029 mmol, 0.05 éq.) de tétrakis(triphénylphosphine)palladium est ajouté. Le mélange est mis au reflux (100°C) pendant 2 heures puis est refroidi à température ambiante. Le milieu réactionnel est extrait deux fois avec de l'acétate d'éthyle. Les phases organiques sont réunies, lavées deux fois avec une solution aqueuse saturée en chlorure de sodium, puis séchées avec du sulfate de magnésium et concentrées. Un solide jaune est alors obtenu. Le brut est purifié sur une colonne de 30 g de silice, avec un gradient d'éluant de 20% à 40% d'acétate d'éthyle dans l'heptane. On obtient un solide jaune (175 mg ).
MS (ES) MH⁺m/z=435.
¹H RMN (DMSO-d6) δ 7.40 (m, 1H), 7.49 (m, 1H), 7.68 (d, J = 8 Hz, 2H), 8.13 (d, J = 8 Hz, 2H), 8.16 (d, J = 8 Hz, 1 H), 8.45 (d, J = 8 Hz, 1 H), 8.56 (m, 1 H).

La 1-(2-fluoro-5-trifluorométhyl-phényl)-3-[4-(4,4,5,5-tétraméthyl-[1,3,2]dioxaborolan-2-yl)-phényl]-urée est préparée selon le mode opératoire suivant :
A une solution de 1g de 4-(4,4,5,5-tétraméthyl-[1,3,2]dioxaborolan-2-yl)-aniline dans 15 ml de tétrahydrofurane on ajoute à température ambiante 936 mg de 2-fluoro-5-trifluorométhyl-phénylisocyanate, puis 0.64 ml de triéthylamine. Le milieu réactionnel est agité à température ambiante pendant 18h, puis traité au méthanol, et enfin évaporé à sec sous pression réduite. Le résidu ainsi obtenu est purifié par chromatographie sur silice en utilisant comme éluant un mélange chlorure de méthylène / méthanol (99.5/0.5 puis 90/10). Les fractions contenant le produit attendu sont concentrées à sec pour donner 1.45g de 1-(2-fluoro-5-trifluorométhyl-phényl)-3-[4-(4,4,5,5-tétraméthyl-[1,3,2]dioxaborolan-2-yl)-phényl]-urée sous la forme d'un solide blanc. MS (ES) MH⁺m/z=425

### Exemple 2

### Thiophene-3-carboxylic acid (6-{4-[3-(2-fluoro-5-trifluoromethyl-phenyl)-ureido]-phenyl}-1H-pyrazolo[3,4-b]pyridin-3-yl)-amide

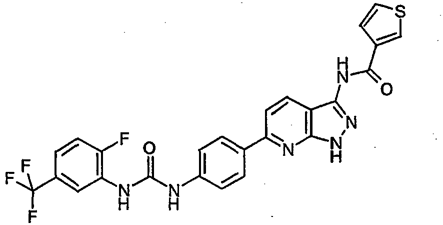

Une solution de 1-[4-(3-Amino-1H-pyrazolo[3,4-b]pyridin-6-yl)-phényl]-3-(2-fluoro-5-trifluorométhyl-phényl)-urée (80.0 mg, 0.186 mmol) dans la pyridine (2 mL) est refroidie à 5°C sous argon et du chlorure de l'acide thiophène-3-carboxylique (27 mg, 0.186 mmol, 1.0 eq.) est ajouté. Le mélange est agité à température ambiante pendant 5,5 h. Un autre équivalent de chlorure de l'acide thiophène-3-carboxylique, (27 mg, 0.186 mmol) est ajouté et le mélange est agité à température ambiante pendant une nuit. La réaction est reprise à l'eau, puis extraite avec de l'acétate d'éthyle. La phase organique est lavée deux fois avec une solution salée, séchée avec du sulfate de magnésium et concentrée. Un solide jaune-orangé est alors obtenu. Le brut est purifié sur une colonne de silice, avec un gradient d'éluant de 0% à 5% de méthanol dans le dichlorométhane. On obtient un solide beige : 13.5 mg. MS (ES) MH⁺m/z=541.
¹H RMN (DMSO-d6) δ 7,40 (m, 1H) ; 7,50 (m, 1H) ; 7,65 (d large, J = 9,0 Hz, 2H) ; 7,68 (dd partiellement masqué, J = 3,0 et 5,0 Hz, 1 H) ; de 7,71 à 7,75 (m, 2H) ; 8,15 (d large, J = 9,0 Hz, 2H) ; 8,39 (d, J = 8,5 Hz, 1 H) ; 8,50 (d large, J = 3,0 Hz, 1H) ; 8,61 (dd, J = 2,5 et 7,5 Hz, 1H) ; 9,21 (m étalé, 1H) ; 9,67 (m étalé, 1 H) ; 10,9 (m étalé, 1 H) ; 13,3 (m étalé, 1H).

### Exemple 3

### N-[4-(3-Amino-1H-pyrazolo[3,4-b]pyridin-6-yl)-phényl]-2,3-dichloro-benzenesulfonamide :

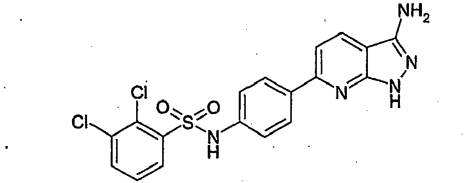

100 mg (0.44 mmol) de 6-(4-Amino-phényl)-1H-pyrazolo[3,4-b]pyridin-3-ylamine est mis en solution dans 3 mL de DMF anhydre. La solution est alors dégazée pendant 15 minutes avec de l'argon. 123.8 µL (89.9 mg, 0.89 mmol, 2 éq.) de triéthylamine est ensuite ajoutée. La solution est encore une fois dégazée 15 minutes. Finalement, 0.109 g (0.444 mmol, 1 éq.) de chlorure de 2,3-dichlorophényl-sulfonyle est ajouté. La solution est agitée à température ambiante sous argon pendant une nuit. En fin de réaction, 10 mL d'eau est ajouté. Un précipité jaune-brun se forme. Le mélange est filtré. Le solide est séché et purifié sur une colonne de 4 g de silice avec un gradient d'éluant de 0 à 10% de méthanol dans le dichlorométhane. Un solide beige est isolé. (11mg).
MS (ES) MH⁺m/z=434.
¹H RMN (DMSO-d6, 373K) δ 5,09 (s large, 2H) ; 7,09 (d large, J = 8,5 Hz, 2H) ; 7,37 (d, J = 8,5 Hz, 1H); 7,40 (t large, J = 8,5 Hz, 1H) ; 7,69 (d large, J = 8,5 Hz, 1H) ; 7,85 (d large, J = 8,5 Hz, 2H) ; 8,02 (d large, J = 8,5 Hz, 1H); 8,06 (d, J = 8,5 Hz, 1H) ; 11,5 (m étalé, 1H).

### Exemple 4

Trifluoroacétate de 6-{2-[3-(2-Fluoro-5-trifluorométhyl-phényl)-ureido]-thiazol-5-yl}-1 H-pyrazolo[3,4-b]pyridine-4-carboxylic acid (2-morpholin-4-yl-ethyl)-amide :

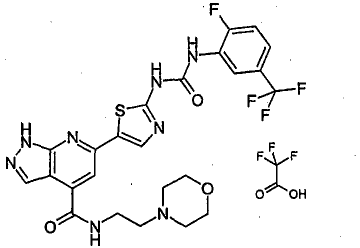

Le produit de l'exemple 4 peut être préparé par le procédé décrit plus haut dans le schéma 4:

### Synthèse de l'intermédiaire 1 :

A une solution de 2-amino-thiazole-5-carboxaldéhyde (2.5g, 19. 5mmol) dans du DMF (120 mL) est ajouté du 2-fluoro-(5-trifluorométhyl)phénylisocyanate (4g, 19.5 mmol). La solution est chauffée à 70°C pendant 1 H, évaporée et purifiée par chromatographie sur gel de silice, éluant hexane/EtOAc 1:1. Les fractions contenant l'intermédiaire 1 sont réunies et évaporées pour fournir l'intermédiaire 1 sous la forme d'un solide jaune. (4.28 g).

### Synthèse de l'intermédiaire 2 :

A une solution de l'intermédiaire 1 (88 mg, 0.26 mmol) dans un mélange éthanol/DMSO 9:1 (2 mL) est ajouté une solution de 1H-pyrazol-3-ylamine (22 mg, 0.26 mmol) dans 2 mL du même mélange de solvants contenant de l'acide pyruvique (23 mg, 0.26 mmol). Le mélange est chauffé à 75°C pendant 24h en présence d'oxygène, puis le milieu réactionnel est évaporé à sec pour fournir l'intermédiaire 2 sous forme d'un solide orange qui est utilisé tel quel à l'étape suivante.

### Préparation du produit de l'exemple 4 :

L'intermédiaire 2 est dissous dans du THF sec (2 mL), du HOBt (36 mg, 0.26 mmol), puis du DIC est ajouté (42 µL, 0.26 mmol). Après 5 min, de la 2-morpholin-4-yl éthylamine (39 µL, 0.3 mmol) est ajoutée. L'agitation est poursuivie pendant 5h à température ambiante, puis le milieu réactionnel est évaporé sous pression réduite. Le résidu est purifié par chromatographie sur gel de silice, éluant EtOAc/MeOH/TEA 94:5:1 en utilisant un gradient de EtOAc/MeOH/TEA 90:9:1. Les fractions contenant le produit sont combinées et évaporées, puis purifiées par chromatographie LC/MS, (phase inverse, éluant eau + 0,1% TFA avec un gradient de 25 à 85% d'acétonitrile sur une période de 8 minutes. Les fractions contenant le produit attendu sont rassemblées et évaporées sous pression réduite pour fournir le produit sous forme d'un solide jaune (10.6 mg).

### Exemple 5

### N-[4-(3-Amino-4-méthylaminocarbonyl-1H-pyrazolo[3,4-b]pyridin-6-yl)-phényl]-2,3-dichloro-benzenesulfonamide :

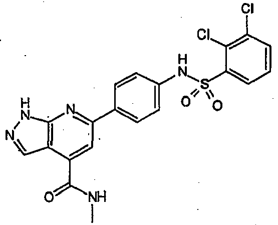

Le produit de l'exemple 5 peut être préparé par le procédé décrit plus haut dans le schéma 5:

### Synthèse de l'intermédiaire 1 :

219 mg de 4-(4,4,5,5-tetraméthyl-1,3,2-dioxaborolan-2-yl)aniline, sont dissous avec 245mg (1mmol) de chlorure de2,3-dichlorobenzènesulfonyle dans 2 ml DCM et 120µl (1.5µmol) de pyridine. Le mélange est agité pendant 6 heures à température ambiante puis séché sous vide pour obtenir le produit désiré sous forme d'un solide rouge clair.
MS (ES) MH⁺m/z=428

### Synthèse de l'intermédiaire 2 :

84 g de sel sodique de diéthyle oxalacétate sont dissous avec 33.2g de 2-amino pyrazole dans 600ml de AcOH/H2O (1:3). Le mélange est chauffé pendant 6 heures à 80°C. Le produit est précipité après refroidissement, filtré et séché sous vide (17.9g).
¹H NMR (600 M Hz, DMSO-d6) δ ppm: 13.74 (s, 1 H), 12.39 (s, 1H), 8.22 (s, 1 H), 6.70 (s, 1 H), 4.40 (q, *J* = 7.1 Hz) 2H), 1.40 (t, *J* = 7.1 Hz) 3H)

### Synthèse de l'intermédiaire 3 :

2.07g (10 mmol) de l'intermédiaire 2, 20ml de toluène et 2.87g (10 mmol) POBr₃ sont chauffés dans un bain d'huile à 110 °C, sous agitation. Le mélange est évaporé et purifié sur une colonne de silice, élué avec 20% AcOEt 80% hexane pour donner (après évaporation) 300mg du produit recherché.
¹H NMR (600 M Hz, DMSO-d6) δ ppm: 14.45 (s, 1 H), 8.41 (s, 1 H), 7.79 (s, 1 H), 4.49 (q, *J* = 7.1 Hz) 2H), 1.46 (t, *J* = 7.1 Hz) 3H)

### Synthèse de l'intermédiaire 4 :

74 µmol de l'intermédiaire 3 (20 mg), 74 µmol de l'intermédiaire 1, 185 µmol de carbonate de césium, et 7.4 µmol. de Pd (PPh₃)₄ sont dissous dans THF et 20% H₂O (750 µl volume total). Le mélange est chauffé au micro-ondes pendant 25 min à 155°C (puissance 55W). Le produit est évaporé et purifié par chromatographie LC/MS dans l'eau avec 0.1 % d'acide trifluoroacétique et un gradient de 25 à 95 % acétonitrile sur 9 minutes. Le produit est séché sous vide MS (ES) MH⁺ m/z=463

### Préparation du produit de l'exemple 5

L'intermédiaire 4 (20mg, 43.3µmol) est dissous dans DMF (0.5 ml), avec HOBt (14.6 mg, 108.25 µmol) et DIC (16.9 µl, 108.25 µmol). 0.5 mmol de méthylamine (1 M dans THF, 0.5 ml) sont ajoutés. Le mélange est chauffé au micro-ondes pendant 20 min à 105°C (puissance 25W). Le produit est évaporé et purifié par chromatographie LC/MS dans l'eau avec 0.1 % d'acide trifluoroacétique et un gradient de 25 à 95 % acétonitrile sur 9 minutes. Le produit est séché sous vide pour obtenir un solide jaune clair.
MS (ES) MH⁺ m/z=476
¹H NMR (600 M Hz, DMSO-d6) δppm: 13.79 (s, 1H), 11.13 (s, 1 H), 8.35 (s, 1 H), 8.02 (s, 1 H), 8.13 (d, *J* = 7.8 Hz) 2H) 8.09 (d, *J* = 8.27 Hz) 1H), 7.94 (d, *J* = 8.05 Hz) 1H), 7.28 (d, *J* = 4.31 Hz) 2H), 2.88 (d, *J* = 4.31 Hz) 3H) 7.58 (q, *J* = 8.08 Hz) 1H), 8.86 (t, *J*=4.43 Hz) 1H)

### Exemples 6 à 62:

En opérant selon les modes opératoires décrits précédemment, on obtient les produits du tableau 1 suivant :

| Exemple | Formule | ESI (MS) (m/z) |
|---|---|---|
| 6 | 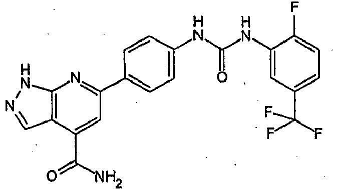 | 459 |
| 7 | 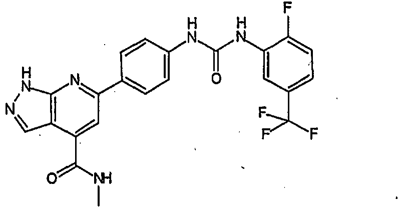 | 473 |
| 8 | 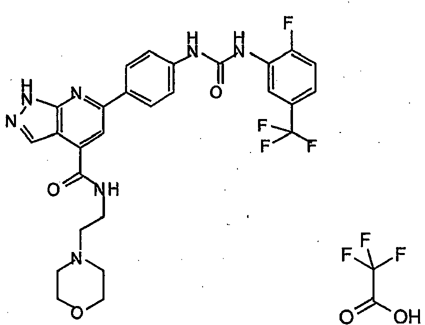 | 573 |
| 9 | 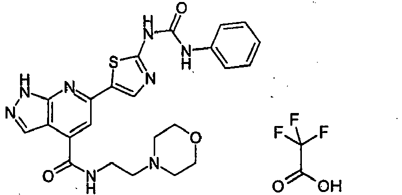 | 494 |
| 10 | 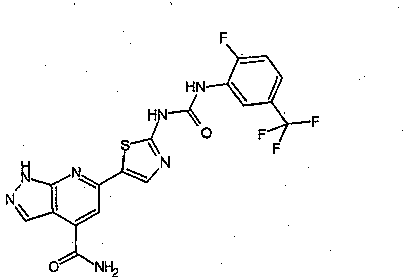 | 466 |
| 11 | 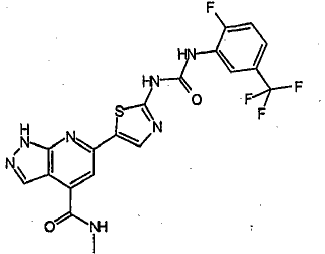 | 480 |
| 12 | 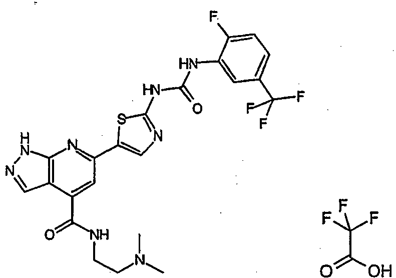 | 538 |
| 13 | 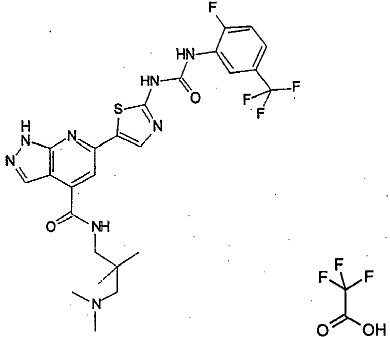 | 580 |
| 14 | 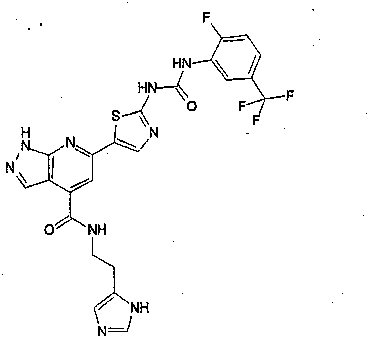 | 561 |
| 15 | 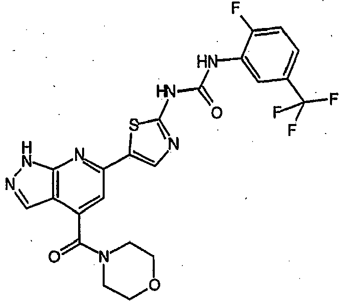 | 536 |
| 16 | 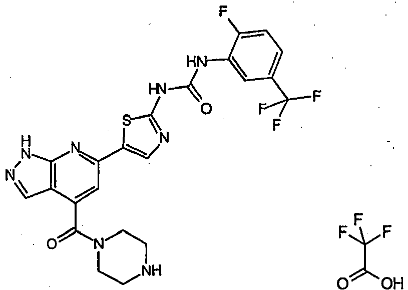 | 535 |
| 17 | 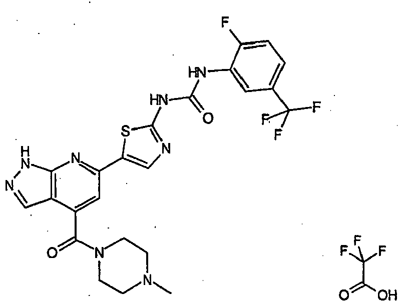 | 550 |
| 18 | 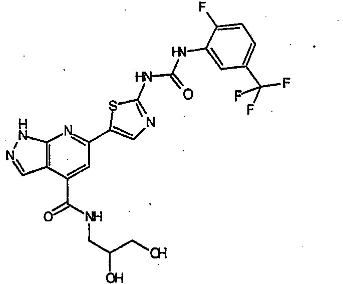 | 540 |
| 19 | 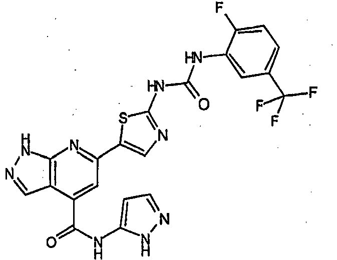 | 532 |
| 20 | 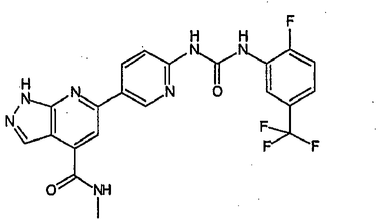 | 474 |
| 21 | 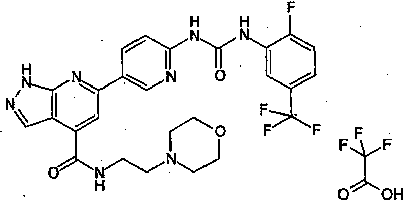 | 574 |
| 22 | 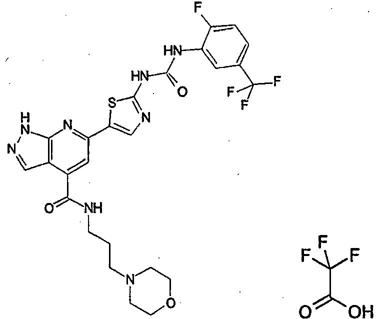 | 594 |
| 23 | 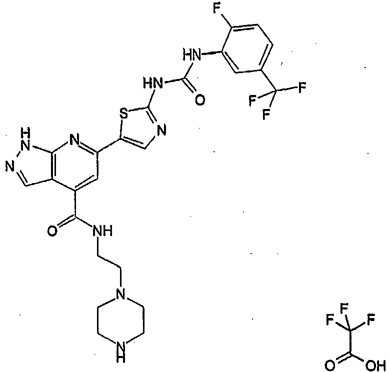 | 579 |
| 24 | 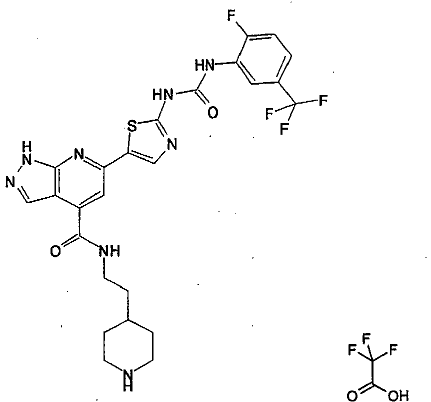 | 578 |
| 25 | 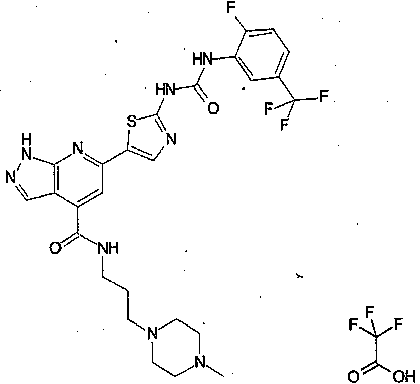 | 607 |
| 26 | 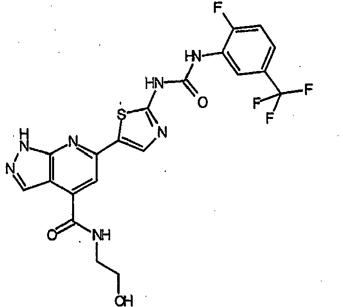 | 510 |
| 27 | 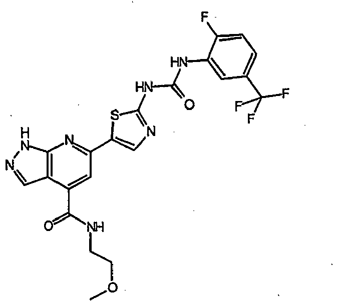 | 524 |
| 28 | 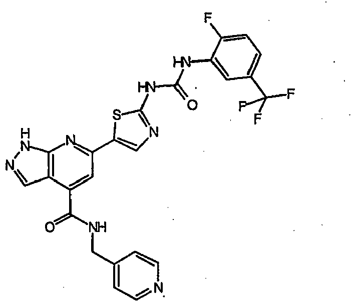 | 558 |
| 29 | 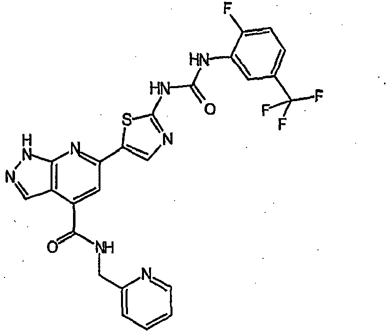 | 558 |
| 30 | 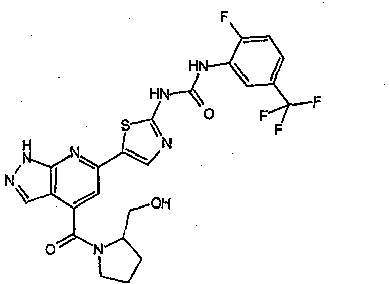 | 551 |
| 31 | 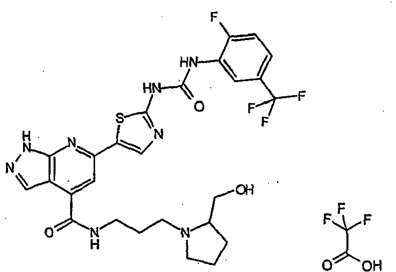 | 608 |
| 32 | 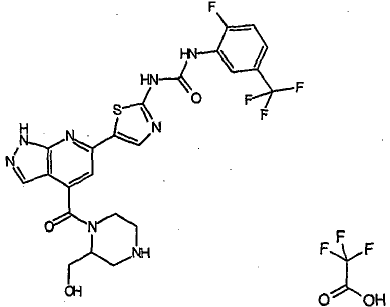 | 566 |
| 33 | 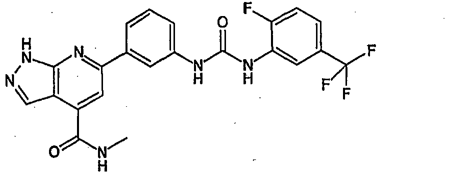 | 473 |
| 34 | 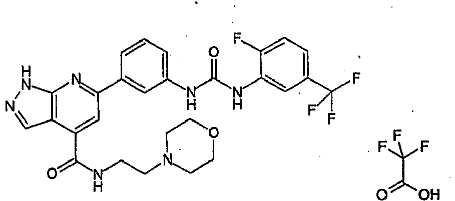 | 573 |
| 35 | 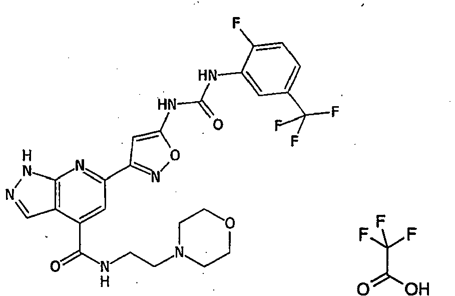 | 563 |
| 36 | 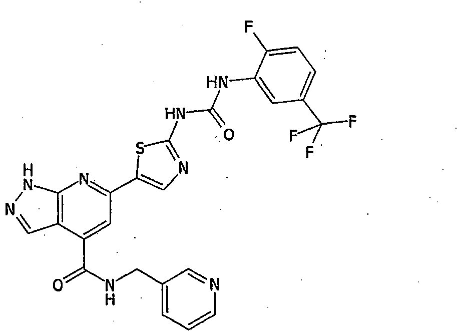 | 558 |
| 37 | 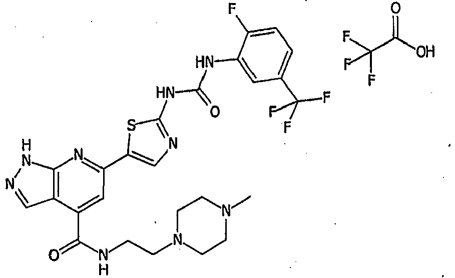 | 591 |
| 38 | 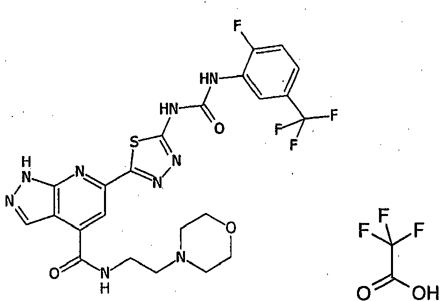 | 581 |
| 39 | 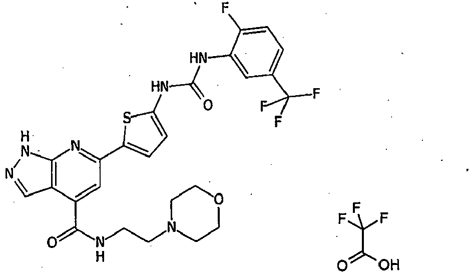 | 579 |
| 40 | 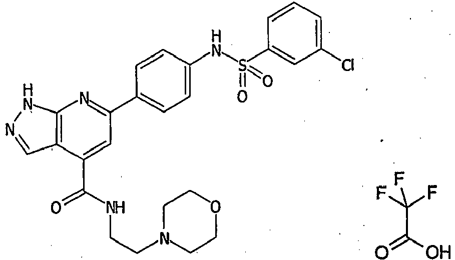 | 542 |
| 41 | 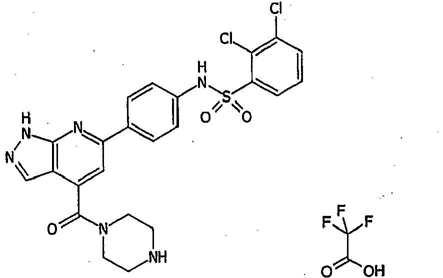 | 532 |
| 42 | 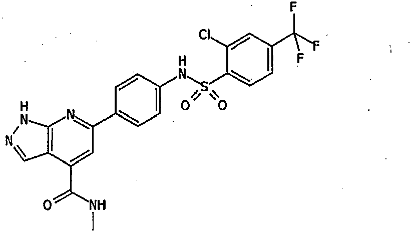 | 511 |
| 43 | 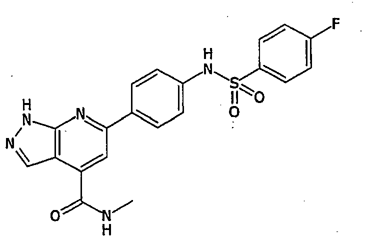 | 426 |
| 44 | 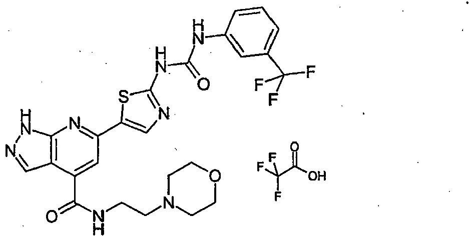 | 561 |
| 45 | 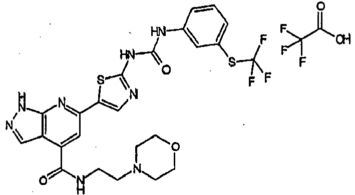 | 593 |
| 46 | 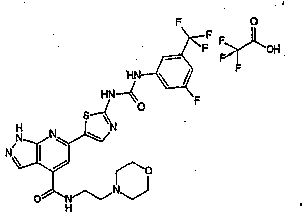 | 579 |
| 47 | 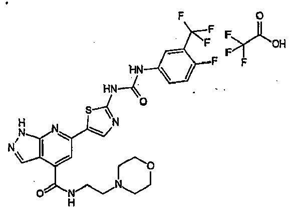 | 579 |
| 48 | 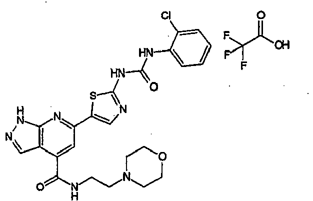 | 527 |
| 49 | 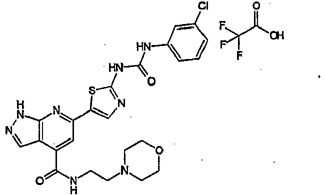 | 527 |
| 50 | 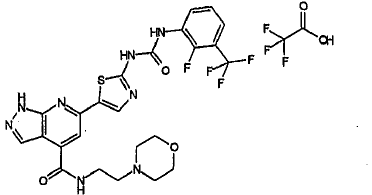 | 579 |
| 51 | 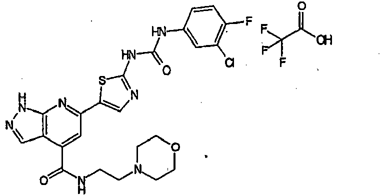 | 545 |
| 52 | 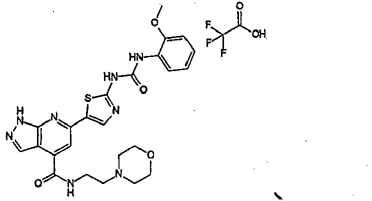 | 523 |
| 53 | 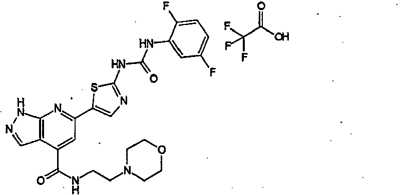 | 529 |
| 54 | 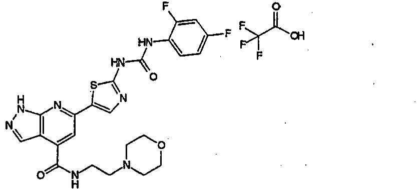 | 529 |
| 55 | 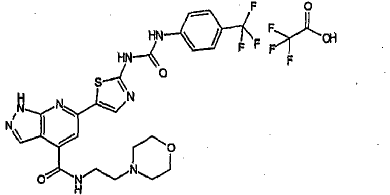 | 561 |
| 56 | 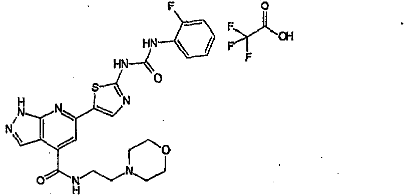 | 511 |
| 57 | 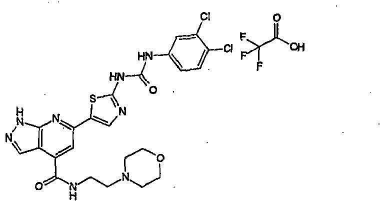 | 561 |
| 58 | 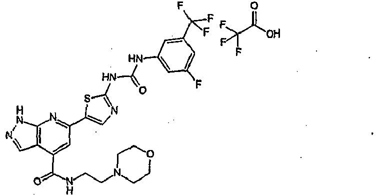 | 577 |
| 59 | 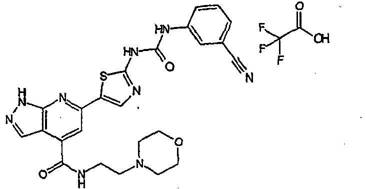 | 518 |
| 60 | 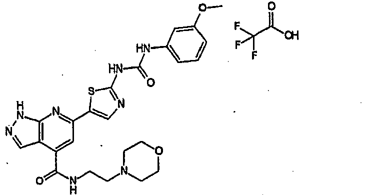 | 523 |
| 61 | 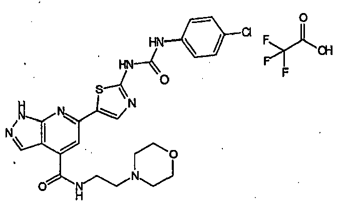 | 527 |
| 62 | 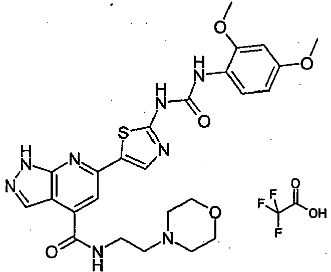 | 553 |

### Détermination de l'activité des composés - Protocoles expérimentaux

### 1. FAK

L'activité inhibitrice des composés sur FAK est déterminée par une mesure de l'inhibition de l'autophosphorylation de l'enzyme en utilisant un test de fluorescence résolue dans le temps (HTRF).

L'ADNc complet de FAK humain, dont l'extrémité N-terminale a été marquée à l'histidine, a été cloné dans un vecteur d'expression baculovirus pFastBac HTc. La protéine a été exprimée et purifiée à environ 70% d'homogénéité.

L'activité kinase est déterminée en incubant l'enzyme (6.6 µg/mL) avec différentes concentrations de composé à tester dans un tampon 50 mM Hepes pH = 7,2, 10 mM MgCl₂, 100 µM Na₃VO₄ ,15 µM d'ATP pendant 1 heure à 37°C. La réaction enzymatique est stoppée par l'addition de tampon Hepes pH = 7,0 contenant 0.4 mM KF, 133 mM EDTA, 0.1 % BSA et le marquage est effectuée, pendant 1 à 2 heures à température ambiante, par l'addition dans ce tampon d'un anticorps anti-Histidine marqué avec XL665 et d'un anticorps monoclonal phosphospécifique de la tyrosine conjugué à du cryptate d'europium (Eu-K). Les caractéristiques des deux fluorophores sont disponibles dans G. Mathis et al., Anticancer Research, 1997, 17, pages 3011-3014. Le transfert d'énergie entre le cryptate d'europium excité vers le XL665 accepteur est proportionnel au degré d'autophosphorylation de FAK. Le signal de longue durée spécifique de XL-665 est mesuré dans un compteur de plaques Packard Discovery. Tous les essais sont effectués en double exemplaire et la moyenne des deux essais est calculée. L'inhibition de l'activité d'autophosphorylation de FAK avec des composés de l'invention est exprimée en pourcentage d'inhibition par rapport à un contrôle dont l'activité est mesurée en l'absence de composé test. Pour le calcul du % d'inhibition, le ratio [signal à 665 nm/signal à 620 nm] est considéré.

### 2. KDR

L'effet inhibiteur des composés est déterminé dans un test de phosphorylation de substrat par l'enzyme KDR *in vitro* par une technique de scintillation (plaque 96 puits, NEN).

Le domaine cytoplasmique de l'enzyme KDR humaine a été cloné sous forme de fusion GST dans le vecteur d'expression baculovirus pFastBac. La protéine a été exprimée dans les cellules SF21 et purifiée à environ 60 % d'homogénéité.

L'activité kinase de KDR est mesurée dans 20 mM MOPS, 10 mM MgCl2, 10 mM MnCl2, 1 mM DTT, 2.5 mM EGTA, 10 mM b-glycérophosphate, pH = 7.2, en présence de 10 mM MgCl₂, 100 µM Na₃VO₄, 1 mM NaF. 10 µl du composé sont ajoutés à 70 µl de tampon kinase contenant 100 ng d'enzyme KDR à 4°C. La réaction est lancée en ajoutant 20 µl de solution contenant 2 µg de substrat (fragment SH2-SH3 de la PLCγ exprimée sous forme de protéine de fusion GST), 2 µCi γ ³³P[ATP] et 2 µM ATP froid. Après 1 heure d'incubation à 37°C, la réaction est stoppée en ajoutant 1 volume (100 µl) de 200 mM EDTA. Le tampon d'incubation est retiré, et les puits sont lavés trois fois avec 300 µl de PBS. La radioactivité est mesurée dans chaque puits en utilisant un compteur de radioactivité Top Count NXT (Packard).

Le bruit de fond est déterminé par la mesure de la radioactivité dans quatre puits différents contenant l'ATP radioactif et le substrat seul.

Un contrôle d'activité totale est mesuré dans quatre puits différents contenant tous les réactifs (γ³³P-[ATP], KDR et substrat PLCγ) mais en l'absence de composé.

L'inhibition de l'activité KDR avec le composé de l'invention est exprimée en pourcentage d'inhibition de l'activité contrôle déterminée en l'absence de composé.

Le composé SU5614 (Calbiochem) (1 µM) est inclus dans chaque plaque comme contrôle d'inhibition.

### 3. Tie2

La séquence codante de Tie2 humain correspondant aux acides aminés du domaine intracellulaire 776-1124 a été générée par PCR en utilisant le cDNA isolé de placenta humain comme modèle. Cette séquence a été introduite dans un vecteur d'expression *baculovirus* pFastBacGT sous forme de protéine de fusion GST.

L'effet inhibiteur des molécules est déterminé dans un test de phosphorylation de PLC par Tie2 en présence de GST-Tie2 purifiée à environ 80% d'homogénéité. Le substrat est composé des fragments SH2-SH3 de la PLC exprimée sous forme de protéine de fusion GST.

L'activité kinase de Tie2 est mesurée dans un tampon MOPS 20mM pH 7.2, contenant 10 mM MgCl₂, 10 mM MnCl₂, 1 mM DTT, 10 mM de glycérophosphate. Dans une plaque 96 puits FlashPlate maintenue sur glace, on dépose un mélange réactionnel composé de 70 µl de tampon kinase contenant 100 ng d'enzyme GST-Tie2 par puits: Ensuite 10 µl de la molécule à tester diluée dans du DMSO à une concentration de 10 % maximum sont ajoutés. Pour une concentration donnée, chaque mesure est effectuée en quatre exemplaires. La réaction est initiée en ajoutant 20 µl de solution contenant 2 µg de GST-PLC, 2 µM d'ATP froid et 1 µCi d'³³P[ATP]. Après 1 heure d'incubation à 37°C, la réaction est stoppée en ajoutant 1 volume (100µl) d'EDTA à 200 mM. Après élimination du tampon d'incubation, les puits sont lavés trois fois avec 300 µl de PBS. La radioactivité est mesurée sur un MicroBeta1450 Wallac.

L'inhibition de l'activité Tie2 est calculée et exprimée en pourcentage d'inhibition par rapport à l'activité contrôle déterminée en l'absence de composé.

| Exemple | IC 50 (nM) | | |
|---|---|---|---|
| | KDR | Tie2 | FAK |
| 1 | 119 | 192 | 10000 |
| 2 | 6 | 7 | |
| 3 | 10000 | 75 | 10000 |
| 4 | 83 | 1 | 343 |
| 5 | 10000 | 13 | 10000 |
| 9 | 6824 | 443 | 10000 |
| 11 | 581 | 8 | 927 |
| 12 | 2360 | 30 | |
| 13 | 373 | 4 | |
| 14 | 293 | 7 | |
| 15 | 70 | 2 | |
| 16 | 200 | 7 | |
| 17 | 46 | 1 | |
| 20 | 10000 | 777 | |
| 21 | 10000 | 6034 | |
| 22 | 511 | 4 | |
| 23 | 527 | 6 | |
| 24 | 133 | 2 | |
| 26 | 387 | 3 | |
| 28 | 4658 | 105 | |
| 29 | 160 | 3 | |
| 31 | 647 | 9 | |
| 33 | 10000 | 2697 | |
| 34 | 10000 | 4144 | |
| 55 | 3538 | 88 | |

## Revendications

1. Produit de formule générale (I) suivante : 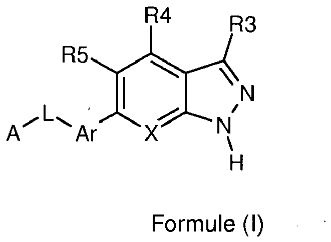 dans laquelle :
1) A et Ar sont indépendamment sélectionnés dans le groupe constitué par : aryle, hétéroaryle, hétérocyclyle, aryle substitué, hétéroaryle substitué, hétérocyclyle substitué, cycloalkyle, et cycloalkyle substitué ;
2) L est sélectionné dans le groupe constitué par : NH, NH-SO₂, SO₂NH, NH-CH₂, CH₂-NH, NH-CO, CO-NH, CH₂-CO-NH, NH-CO-CH₂, NH-CH₂-CO, CO-CH₂-NH, NH-CO-NH, NH-CS-NH, NH-CO-O, O-CO-NH, CH₂-NH-CO-NH, NH-CO-NH-CH₂, et NH-CO-CH₂-CO-NH;
3) X est N ou NO ;
4) R3 est sélectionné dans le groupe constitué par H et NHMR"3, dans lequel M est sélectionné dans le groupe constitué par : liaison, CO, CO-NH, CS, CS-NH, SO₂ ; et dans lequel R"3 est sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, et hétérocyclyle substitué ;
5) R4 est sélectionné dans le groupe constitué par : H, halogène, alkyle, alkyle substitué, OR"4 , N(R"5)(R"6), CON(R"5)(R"6), dans lequel R"4 est choisi parmi H, phényle, phényle substitué, alkyle, alkyle substitué, et dans lequel R"5 et R"6 sont indépendamment sélectionnés dans le groupe constitué par H, (C1-C6)alkyle, (C1-C6)alkyle substitué, -(C1-C6)alkyle-hétérocyclyle, -(C1-C6)alkyle-hétérocyclyle substitué, -(C1-C6)alkyle-hétéroaryle, -(C1-C6)alkyle-hétéroaryle substitué, cycloalkyle, cycloalkyle substitué, hétérocyclyle, hétérocyclyle substitué, aryle, aryle substitué, hétéroaryle, hétéroaryle substitué ou bien R"5 et R"6 sont liés entre eux pour former un cycle saturé de 4 à 8 chaînons contenant de 1 à 3 hétéroatomes choisis parmi O, S et N, éventuellement substitué;
6) R5 est sélectionné dans le groupe constitué par : H, halogène, R'2, CN, O(R'2), OC(O)(R'2), OC(O)N(R'2)(R'3), OS(O₂)(R'2), N(R'2)(R'3), N=C(R'₂)(R'₃), N(R'2)C(O)(R'3), N(R'2)C(O)O(R'3), N(R'4)C(O)N(R'2)(R'3), N(R'4)C(S)N(R'2)(R'3), N(R'2)S(O₂)(R'3), C(O)(R'2), C(O)O(R'2), C(O)N(R'2)(R'3), C(=N(R'3))(R'2), C(=N(OR'3))(R'2), S(R'2), S(O)(R'2), S(O₂)(R'2), S(O₂)O(R'2), S(O₂)N(R'2)(R'3) ; dans lequel chaque R'2, R'3, R'4 est indépendamment sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, hétérocyclyle substitué; et R'2 et R'3 peuvent être liés entre eux pour former un cycle contenant de 1 à 3 hétéroatomes choisis parmi O, S et N ;
À la condition que lorsque X est N, R3 est NH₂, Ar et A sont phényle non substitué, L est NHCO lié en para de Ar, et R5 est H, alors R4 n'est pas choisi parmi : phényle, *o*-chlorophényle, cinnamyle, α-furfuryle, *o*-hydroxyphényle, *p-*hydroxy-*m*-méthoxyphényle, *p*-méthylthiophényle, *p*-méthoxyphényle, *o-*nitrophényle, *m*-phénoxyphényle, et à la condition que lorsque X est N, R5 est H, R4 est H, et Ar-L-A est un groupe 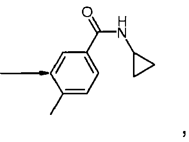 alors R3 n'est pas choisi parmi : amino, acétylamino, [(4-fluorophényl)carbonyl]amino, (2-méthylpropanoyl)amino, -(cyclopentylcarbonyl)amino, propanoylamino, [(4-méthylphényl)carbonyl]amino, {[4-(methyloxy)phenyl]carbonyl}amino, (2-thiénylcarbonyl)amino, (méthylsulfonyl)amino, -[(4-fluorophényl)sulfonyl]amino, (éthylsulfonyl)amino, (propylsulfonyl)amino, (3-thiénylsulfonyl)amino, [(3,5-diméthyl-4-isoxazolyl)sulfonyl]amino, (2-thiénylsulfonyl)amino, (1-méthyléthyl)amino.

2. Produit selon la revendication 1, **caractérisé en ce que** :
1) A et Ar sont indépendamment sélectionnés dans le groupe constitué par : aryle, hétéroaryle, hétérocyclyle, cycloalkyle, aryle substitué, hétéroaryle substitué, hétérocyclyle substitué, et cycloalkyle substitué ;
2) L est sélectionné dans le groupe constitué par : NH, NH-SO₂, SO₂NH, NH-CH₂, CH₂-NH, CH₂-CO-NH, NH-CO-CH₂, NH-CH₂-CO, CO-CH₂-NH, NH-CO-NH, NH-CS-NH, NH-CO-O, O-CO-NH, CH₂-NH-.CO-NH, NH-CO-NH-CH₂, et NH-CO-CH₂-CO-NH;
3) X est N ;
4) R3 est sélectionné parmi H , NH2 et NHCOR"3, dans lequel R"3 est sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, et hétérocyclyle substitué ;
5) R4 est sélectionné dans le groupe constitué par : H, halogène, alkyle, alkyle substitué, CON(R"5)(R"6) dans lequel R"5 et R"6 sont indépendamment sélectionnés dans le groupe constitué par H, (C1-C6)alkyle, (C1-C6)alkyle substitué, -(C1-C6)alkyle-hétérocyclyle, -(C1-C6)alkyle-hétérocyclyle substitué, - (C1-C6)alkyle-hétéroaryle, -(C1-C6)alkyle-hétéroaryle substitué, cycloalkyle, cycloalkyle substitué, hétérocyclyle, hétérocyclyle substitué, aryle, aryle substitué, hétéroaryle, hétéroaryle substitué ou bien R"5 et R"6 sont liés entre eux pour former un cycle saturé de 4 à 8 chaînons contenant de 1 à 3 hétéroatomes choisis parmi O, S et N, éventuellement substitué ;
6) R5 est H.

3. Produit selon la revendication 1 ou 2, **caractérisé en ce que** Ar est choisi dans le groupe constitué par : thiazolyle, thiényle, furyle, pyrrolyle, oxazolyle, isoxazolyle, isothiazolyle, thiadiazolyle, pyrazolyle, imidazolyle, indolyle, indazolyle, benzimidazolyle, benzoxazolyle, et benzothiazolyle ; éventuellement substitué.

4. Produit selon la revendication 1 ou 2, **caractérisé en ce que** Ar-L-A est : 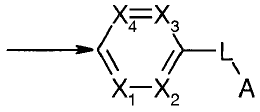
dans lequel chaque X1, X2, X3 et X4 est indépendamment choisi parmi N et C-R'5, dans lequel R'5 a la même définition que R5.

5. Produit selon la revendication 1, **caractérisé en ce que** L-A est sélectionné dans le groupe constitué par NH-CO-NH-A et NH-SO₂-A.

6. Produit selon la revendication 1, **caractérisé en ce que** A est sélectionné dans le groupe constitué par : phényle, pyridyle, pyrimidyle, thiényle, furyle, pyrrolyle, oxazolyle, thiazolyle, isoxazolyle, isothiazolyle, pyrazolyle, imidazolyle, indolyle, indazolyle, benzimidazolyle, benzoxazolyle, et benzothiazolyle ; éventuellement substitué.

7. Produit selon la revendication 6, **caractérisé en ce que** A est sélectionné dans le groupe constitué par : phényle, pyrazolyle et isoxazolyle ; éventuellement substitué.

8. Produit selon la revendication 1, **caractérisé en ce que** A est substitué par un premier substituant sélectionné dans le groupe constitué par halogène, alkyle, alkylène, alkynyle, aryle, hétéroaryle, O-alkyle, O-Aryle, O-hétéroaryle, S-alkyle, S-Aryle, S-hétéroaryle, chacun étant éventuellement substitué par un ou plusieurs substituant choisi parmi (C1-C3)alkyle, halogène, O-(C1-C3)alkyle.

9. Produit selon la revendication 8, **caractérisé en ce que** A est substitué par un deuxième substituant sélectionné dans le groupe constitué par F, Cl, Br, I, OH, SH, SO₃M, COOM, CN, NO₂, CON(R8)(R9), N(R8)CO(R9), (C1-C3)alkyle-OH; (C1-C3)alkyle-N(R8)(R9), (C1-C3)alkyle-(R10), (C1-C3)alkyle-COOH, N(R8)(R9) ; dans lequel R8 et R9 sont indépendamment choisis parmi H, (C1-C3)alkyle, (C1-C3)alkyle halogéné, (C1-C3)alkyleOH, (C1-C3)alkyle-O(C1-C3)alkyle, (C1-C3)alkyleNH₂, (C1-C3)alkyleN(R8)(R9), (C1-C3)alkyleCOOM, (C1-C3)alkyleSO₃M ; dans lequel lorsque R8 et R9 sont simultanément différents de H, ils peuvent être liés pour former un cycle de 5 à 7 chaînons contenant de 1 à 3 hétéroatomes ; dans lequel M est H ou un cation de métal alcalin choisi parmi Li, Na et K ; et dans lequel R10 est H ou un hétérocycle non aromatique éventuellement substitué, comprenant 2 à 7 atomes de carbone, et 1 à 3 hétéroatomes choisis parmi N, O et S.

10. Produit selon la revendication 1, **caractérisé en ce que** A est choisi parmi phényle, pyrazolyle et isoxazolyle ; éventuellement substitué par halogène, (C1-C4)alkyle, (C1-C3)alkyle halogéné, O-(C1-C4)alkyle, S-(C1-C4)alkyle, O-(C1-C4)alkyle halogéné, et S-(C1-C4)alkyle halogéné, et **en ce que**, lorsque A est disubstitué, les deux substituants de A peuvent former un cycle de 5 à 7 chaînons contenant de 0 à 3 hétéroatomes choisis parmi O, N et S.

11. Produit selon la revendication 1, **caractérisé en ce que** R4 est H ou CON(R"5)(R"6), avec R"5 et R"6 tels que définis précédemment.

12. Produit selon l'une quelconque des revendications 1 à 11 **caractérisé en ce qu'**il est choisi parmi :
1-[4-(3-Amino-1H-pyrazolo[3,4-b]pyridin-6-yl)-phényl]-3-(2-fluoro-5-trifluorométhyl-phényl)-urée ;
Thiophene-3-carboxylic acid (6-{4-[3-(2-fluoro-5-trifluorométhyl-phényl)-uréido]-phényl}-1*H*-pyrazolo[3,4-*b*]pyridin-3-yl)-amide ;
Trifluoroacétate de 6-{2-[3-(2-Fluoro-5-trifluorométhyl-phényl)-uréido]-thiazol-5-yl}-1 H-pyrazolo[3,4-b]pyridine-4-carboxylic acid (2-morpholin-4-yl-éthyl)-amide ;
6-{4-[3-(2-Fluoro-5-trifluorométhyl-phényl)-uréido]-phényl}-1 H-pyrazolo[3,4-.
b]pyridine-4-carboxylic acid amide ;
6-{4-[3-(2-Fluoro-5-trifluorométhyl-phényl)-uréido]-phényl}-1H-pyrazolo[3,4-b]pyridine-4-carboxylic acid méthylamide ;
6-{4-[3-(2-Fluoro-5-trifluorométhyl-phényl)-uréido]-phényl}-1H-pyrazolo[3,4-b]pyridine-4-carboxylic acid (2-morpholin-4-yl-éthyl)-amide ;
6-[2-(3-Phényl-uréido)-thiazol-5-yl]-1 H-pyrazolo[3,4-b]pyridine-4-carboxylic acid (2-morpholin-4-yl-éthyl)-amide ;
6-{2-[3-(2-Fluoro-5-trifluorométhyl-phényl)-uréido]-thiazol-5-yl}-1H-pyrazolo[3,4-b]pyridine-4-carboxylicacid amide ;
6-{2-[3-(2-Fluoro-5-trifluorométhyl-phényl)-uréido]-thiazol-5-yl}-1H-pyrazolo[3,4-b]pyridine-4-carboxylicacid méthylamide ;
6-{2-[3-(2-Fluoro-5-trifluorométhyl-phényl)-uréido]-thiazol-5-yl}-1H-pyrazolo[3,4-b]pyridine-4-carboxylicacid (2-diméthylamino-éthyl)-amide ;
6-{2-[3-(2-Fluoro-5-trifluorométhyl-phényl)-uréido]-thiazol-5-yl}-1H-pyrazolo[3,4-b]pyridine-4-carboxylicacid (3-diméthylamino-2,2-diméthy-propyl)amide ;
6-{2-[3-(2-Fluoro-5-trifluorométhyl-phényl)-uréido]-thiazol-5-yl}-1H-pyrazolo[3,4-b]pyridine-4-carboxylicacid [2-(3H-imidazol-4-yl)-éthyl]-amide ;
1-((2-Fluoro-5-trifluorométhyl-phényl)-3-{5-[4-(morpholine-4-carbonyl)-1H-pyrazolo[3,4-b]pyridin-6-yl]-thiazol-2-yl}-urée ;
1-((2-Fluoro-5-trifluorométhyl-phényl)-3-{5-[4-(pipérazine-1-carbonyl)-1H-pyrazolo[3,4-b]pyridin-6-yl]-thiazol-2-yl}-urée ;
1-((2-Fluoro-5-trifluorométhyl-phényl)-3-{5-[4-(4-méthyl-pipérazine-1-carbonyl)-1H-pyrazolo[3,4-b]pyridin-6-yl]-thiazol-2-yl}-urée ;
6-{2-[3-(2-Fluoro-5-trifluorométhyl-phényl)-uréido]-thiazol-5-yl}-1H-pyrazolo[3,4-b]pyridine-4-carboxylic acid (2,3-dihydroxy-propyl)-amide ;
6-{2-[3-(2-Fluoro-5-trifluorométhyl-phényl)-uréido]-thiazol-5-yl}-1H-pyrazolo[3,4-b]pyridine-4-carboxylicacid (2H-pyrazol-3-yl)-amide ;
6-{6-[3-(2-Fluoro-5-trifluorométhyl-phényl)-uréido]-pyridin-3-yl}-1H-pyrazolo[3,4-b]pyridine-4-carboxylicacid méthylamide ;
6-{6-[3-(2-Fluoro-5-trifluorométhyl-phényl)-uréido]-pyridin-3-yl}-1H-pyrazolo[3,4-b]pyridine-4-carboxylic acid (2-morpholin-4-yl-éthyl)-amide ;
6-{2-[3-(2-Fluoro-5-trifluorométhyl-phényl)-uréido]-thiazol-5-yl}-1H-pyrazolo[3,4-b]pyridine-4-carboxylic acid (3-morpholin-4-yl-propyl)-amide ;
6-{2-[3-(2-Fluoro-5-trifluorométhyl-phényl)-uréido]-thiazol-5-yl}-1H-pyrazolo[3,4-b]pyridine-4-carboxylic acid (2-pipérazin-1-yl-éthyl)-amide ;
6-{2-[3-(2-Fluoro-5-trifluorométhyl-phényl)-uréido]-thiazol-5-yl}-1H-pyrazolo[3,4-b]pyridine-4-carboxylic acid (2-pipéridin-4-yl-éthyl)-amide ;
6-{2-[3-(2-Fluoro-5-trifluorométhyl-phényl)-uréido]-thiazol-5-yl}-1H-pyrazolo[3,4-b]pyridine-4-carboxylic acid [3-(4-méthyl-pipérazin-1-yl)-propyl]amide ;
6-{2-[3-(2-Fluoro-5-trifluorométhyl-phényl)-uréido]-thiazol-5-yl}-1H-pyrazolo[3,4-b]pyridine-4-carboxylic acid (2-hydroxy-éthyl)-amide ;
6-{2-[3-(2-Fluoro-5-trifluorométhyl-phényl)-uréido]-thiazol-5-yl}-1H-pyrazolo[3,4-b]pyridine-4-carboxylicacid (2-méthoxy-éthyl)-amide ;
6-{2-[3-(2-Fluoro-5-trifluorométhyl-phényl)-uréido]-thiazol-5-yl}-1H-pyrazolo(3,4-b]pyridine-4-carboxylic acid (pyridin-4-ylméthyl)-amide ;
6-{2-[3-(2-Fluoro-5-trifluorométhyl-phényl)-uréido]-thiazol-5-yl}-1H-pyrazolo[3,4-b]pyridine-4-carboxylic acid (pyridin-2-ylméthyl)-amide ;
1-((2-Fluoro-5-trifluorométhyl-phényl)-3-{5-[4-(2-hydroxyméthyl-pyrrolidine-1-carbonyl)-1H-pyrazolo[3,4-b]pyridin-6-yl]-thiazol-2-yl}-urée ;
6-{2-[3-(2-Fluoro-5-trifluorométhyl-phényl)-uréido]-thiazol-5-yl}-1H-pyrazolo[3,4-b]pyridine-4-carboxylic acid [3-(2-hydroxyméthyl-pyrrolidin-1-yl)propyl]-amide ;
1-((2-Fluoro-5-trifluorométhyl-phényl)-3-{5-[4-(2-hydroxyméthyl-pipérazine-1-carbonyl)-1 H-pyrazolo[3,4-b]pyridin-6-yl]-thiazol-2-yl}-uréa ;
6-{3-[3-(2-Fluoro-5-trifluorométhyl-phényl)-uréido]-phényl}-1 H-pyrazolo[3,4-b]pyridine-4-carboxylic acid méthylamide ;
6-{3-[3-(2-Fluoro-5-trifluorométhyl-phényl)-uréido]-phényl}-1 H-pyrazolo[3,4-b]pyridine-4-carboxylic acid (2-morpholin-4-yl-éthyl)-amide ;
6-{5-[3-(2-Fluoro-5-trifluorométhyl-phényl)-uréido]-isoxazol-3-yl}-1 H-pyrazolo[3,4-b]pyridine-4-carboxylic acid (2-morpholin-4-yl-éthyl)-amide ;
6-{2-[3-(2-Fluoro-5-trifluorométhyl-phényl)-uréido]-thiazol-5-yl}-1 H-pyrazolo[3,4-b]pyridine-4-carboxylicacid (pyridin-3-ylméthyl)-amide ;
6-{2-[3-(2-Fluoro-5-trifluorométhyl-phényl)-uréido]-thiazol-5-yl}-1 H-pyrazolo[3,4-b]pyridine-4-carboxylic acid [2-(4-méthyl-pipérazin-1-yl)-éthyl]-amide ;
6-{5-[3-(2-Fluoro-5-trifluorométhyl-phényl)-uréido]-[1,3,4]thiadiazol-2-yl}-1 H-pyrazolo[3,4-b]pyridine-4-carboxylic acid (2-morpholin-4-yl-éthyl)-amide ;
6-{5-[3-(2-Fluoro-5-trifluorométhyl-phényl)-uréido]-thiophen-2-yl}-1H-pyrazolo[3,4-b]pyridine-4-carboxylic acid (2-morpholin-4-yl-éthyl)-amide ;
6-{2-[3-(3-Trifluorométhyl-phényl)-uréido]-thiazol-5-yl}-1 H-pyrazolo[3,4-b]pyridine-4-carboxylic acid (2-morpholin-4-yl-éthyl)-amide ;
6-{2-[3-(3-Trifluorométhylsulfanyl-phényl)-uréido]-thiazol-5-yl}-1 H-pyrazolo[3,4-b]pyridine-4-carboxylic acid (2-morpholin-4-yl-éthyl)-amide ;
6-{2-[3-(3-Fluoro-5-trifluorométhyl-phényl)-uréido]-thiazol-5-yl}-1H-pyrazolo[3,4-b]pyridine-4-carboxylic acid (2-morpholin-4-yl-éthyl)-amide ;
6-{2-[3-(4-Fluoro-3-trifluorométhyl-phényl)-uréido]-thiazol-5-yl}-1H-pyrazolo[3,4-b]pyridine-4-carboxylic acid (2-morpholin-4-yl-éthyl)-amide ;
6-{2-[3-(2-Chloro-phényl)-uréido]-thiazol-5-yl}-1H-pyrazolo[3,4-b]pyridine-4-carboxylic acid (2-morpholin-4-yl-éthyl)-amide ;
6-{2-[3-(3-Chloro-phényl)-uréido]-thiazol-5-yl}-1H-pyrazolo[3,4-b]pyridine-4-carboxylic acid (2-morpholin-4-yl-éthyl)-amide ;
6-{2-[3-(2-Fluoro-3-trifluorométhyl-phényl)-uréido]-thiazol-5-yl}-1H-pyrazolo[3,4-b]pyridine-4-carboxylic acid (2-morpholin-4-yl-éthyl)-amide ;
6-{2-[3-(3-Chloro-4-fluoro-phényl)-uréido]-thiazol-5-yl}-1H-pyrazolo[3,4-b]pyridine-4-carboxylic acid (2-morpholin-4-yl-éthyl)-amide ;
6-{2-[3-(2-Méthoxy-phényl)-uréido]-thiazol-5-yl}-1H-pyrazolo[3,4-b]pyridine-4-carboxylic acid (2-morpholin-4-yl-éthyl)-amide ;
6-{2-[3-(2,5-Difluoro-phényl)-uréido]-thiazol-5-yl}-1H-pyrazolo[3,4-b]pyridine-4-carboxylic acid (2-morpholin-4-yl-éthyl)-amide ;
6-{2-[3-(2,4-Difluoro-phényl)-uréido]-thiazol-5-yl}-1H-pyrazolo[3,4-b]pyridine-4-carboxylic acid (2-morpholin-4-yl-éthyl)-amide ;
6-{2-[3-(4-Trifluorométhyl-phényl)-uréido]-thiazol-5-yl}-1H-pyrazolo[3,4-b]pyridine-4-carboxylic acid (2-morpholin-4-yl-éthyl)-amide ;
6-{2-[3-(2-Fluoro-phényl)-uréido]-thiazol-5-yl}-1H-pyrazolo[3,4-b]pyridine-4-carboxylic acid (2-morpholin-4-yl-éthyl)-amide ;
6-{2-[3-(3,4-Dichloro-phényl)-uréido]-thiazol-5-yl}-1H-pyrazolo[3,4-b]pyridine-4-carboxylic acid (2-morpholin-4-yl-éthyl)-amide ;
6-{2-[3-(4-Trifluorométhoxy-phényl)-ureido]-thiazol-5-yl}-1H-pyrazolo[3,4-b]pyridine-4-carboxylic acid (2-morpholin-4-yl-éthyl)-amide ;
6-{2-[3-(3-Cyano-phényl)-uréido]-thiazol-5-yl}-1H-pyrazolo[3,4-b]pyridine-4-carboxylic acid (2-morpholin-4-yl-éthyl)-amide ;
6-{2-[3-(3-Méthoxy-phényl)-uréido]-thiazol-5-yl}-1H-pyrazolo[3,4-b]pyridine-4-carboxylic acid (2-morpholin-4-yl-éthyl)-amide ;
6-{2-[3-(4-Chloro-phényl)-uréido]-thiazol-5-yl}-1H-pyrazolo[3,4-b]pyridine-4-carboxylic acid (2-morpholin-4-yl-éthyl)-amide ;
6-{2-[3-(2,4-Diméhoxy-phényl)-uréido]-thiazol-5-yl}-1H-pyrazolo[3,4-b]pyridine-4-carboxylic acid (2-morpholin-4-yl-éthyl)-amide.

13. Produit selon l'une quelconque des revendications 1 à 11 **caractérisé en ce qu'**il est choisi parmi :
N-[4-(4-(2-morpholin-4-yl-éthyl)aminocarbonyl-1H-pyrazolo[3,4-b]pyridin-6-yl)-phényl]- 3-chloro-benzenesulfonamide ;
N-[4-(4-(pipérazine-1-carbonyl)-1H-pyrazolo[3,4-b]pyridin-6-yl)-phényl]-2,3-dichloro-benzenesulfonamide ;
N-[4-(4-méthylaminocarbonyl-1H-pyrazolo[3,4-b]pyridin-6-yl)-phényl]-2-chloro-4-trifluorométhyl-benzenesulfonamide ;
N-[4-(4-méthylaminocarbonyl-1H-pyrazolo[3,4-b]pyridin-6-yl)-phényl]-4-fluoro-benzenesulfonamide ;
N-[4-(3-Amino-1 H-pyrazolo[3,4-b]pyridin-6-yl)-phényl]-2,3-dichloro-benzenesulfonamide ;
N-[4-(3-Amino-4-méthylaminocarbonyl-1H-pyrazolo[3,4-b]pyridin-6-yl)-phényl]-2,3-dichloro-benzenesulfonamide ;

14. Produit selon l'une quelconque des revendications précédentes, **caractérisé en ce qu'**il est sous forme :
a) non chirale, ou
b) racémique, ou
c) enrichie en un stéréo-isomère, ou
d) enrichie en un énantiomère ;
et **en ce qu'**il est éventuellement salifié.

15. Médicament, **caractérisé en ce qu'**il comprend un produit de formule (I) selon l'une quelconque des revendication 1 à 14, ou un sel d'addition de ce composé à un acide pharmaceutiquement acceptable, ou encore un hydrate ou un solvate du produit de formule (I).

16. Composition pharmaceutique comprenant un produit selon l'une quelconque des revendications précédentes, en combinaison avec un excipient pharmaceutiquement acceptable.

17. Produit selon l'une quelconque des revendications 1 à 14 pour son utilisation comme agent inhibiteur d'une réaction catalysée par une ou plusieurs kinases.

18. Produit pour son utilisation selon la revendication 17, **caractérisée en ce que** la kinase est choisie parmi FAK, KDR et Tie2.

19. Utilisation d'un produit selon l'une quelconque des revendications 1 à 14, pour la fabrication d'un médicament utile pour traiter un état pathologique.

20. Utilisation selon la revendication 19, **caractérisée en ce que** l'état pathologique est le cancer.

21. Utilisation selon la revendication 19 ou 20, **caractérisée en ce que** l'état pathologique est choisi parmi l'arthrite rhumatoïde, l'osthéoarthrite et/ou ses douleurs associées, les maladies inflammatoires de l'intestin telle que la recto-colite hémorragique ou la maladie de Crohn's, les pathologies de l'oeil telle que la dégénérescence maculaire liée à l'âge, les rétinopathies diabétiques, l'inflammation chronique, le psoriasis, et des cancers particuliers tels que Je sarcome de Kaposi ou l'hémoangiome infantile.

22. Utilisation, selon les revendications 19 à 21, d'un produit selon l'une quelconque des revendications 1 à 14, pour le traitement ou la prévention d'un état pathologique **caractérisé en ce que** le produit est administré seul ou en association avec d'autres principes actifs notamment des anti-cancéreux tels que des produits cytotoxiques, cytostatiques, anti-angiogéniques, ou anti-métastasiques.

## Patentansprüche

1. Produkt der folgenden allgemeinen Formel (I): 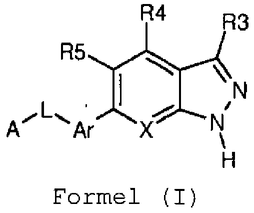 worin:
1) A und Ar unabhängig ausgewählt sind aus der Gruppe bestehend aus: Aryl, Heteroaryl, Heterocyclyl, substituiertem Aryl, substituiertem Heteroaryl, substituiertem Heterocyclyl, Cycloalkyl und substituiertem Cycloalkyl;
2) L ausgewählt ist aus der Gruppe bestehend aus: NH, NH-SO₂, SO₂NH, NH-CH₂, CH₂-NH, NH-CO, CO-NH, CH₂-CO-NH, NH-CO-CH₂, NH-CH₂-CO, CO-CH₂-NH, NH-CO-NH, NH-CS-NH, NH-CO-O, O-CO-NH, CH₂-NH-CO-NH, NH-CO-NH-CH₂ und NH-CO-CH₂-CO-NH;
3) X für N oder NO steht;
4) R3 ausgewählt ist aus der Gruppe bestehend aus H und NHMR''3, worin M ausgewählt ist aus der Gruppe bestehend aus einer Bindung, CO, CO-NH, CS, CS-NH und SO₂ und worin R''3 ausgewählt ist aus der Gruppe bestehend aus H, Alkyl, Alkylen, Alkinyl, Aryl, Heteroaryl, Cycloalkyl, Heterocyclyl, substituiertem Alkyl, substituiertem Alkylen, substituiertem Alkinyl, substituiertem Aryl, substituiertem Heteroaryl, substituiertem Cycloalkyl und substituiertem Heterocyclyl;
5) R4 ausgewählt ist aus der Gruppe bestehend aus: H, Halogen, Alkyl, substituiertem Alkyl, OR''4, N(R"5) (R''6), CON(R''5) (R''6), worin R''4 ausgewählt ist aus H, Phenyl, substituiertem Phenyl, Alkyl und substituiertem Alkyl und worin R''5 und R''6 unabhängig ausgewählt sind aus der Gruppe bestehend aus H, (C₁-C₆)Alkyl, substituiertem (C₁-C₆)Alkyl, -(C₁-C₆)Alkylheterocyclyl, substituiertem -(C₁-C₆)Alkylheterocyclyl, -(C₁-C₆) Alkylheteroaryl, substituiertem -(C₁-C₆)Alkylheteroaryl, Cycloalkyl, substituiertem Cycloalkyl, Heterocyclyl, substituiertem Heterocyclyl, Aryl, substituiertem Aryl, Heteroaryl und substituiertem Heteroaryl oder R''5 und R''6 unter Bildung eines 4- bis 8-gliedrigen gesättigten Rings, der 1 bis 3 aus O, S und N ausgewählte Heteroatome enthält und gegebenenfalls substituiert ist, miteinander verbunden sind;
6) R5 ausgewählt ist aus der Gruppe bestehend aus: H, Halogen, R'2, CN, O(R'2), OC(O)(R'2), OC(O)N(R'2)(R'3), OS(O₂) (R'2), N(R'2)(R'3), N=C(R'2)(R'3), N(R'2)C(O)(R'3), N(R'2)C(O)O(R'3), N(R'4)C(0)N(R'2) (R'3), N(R'4)C(S)N(R'2)(R'3), N(R'2)S(O₂) (R'3), C(O)(R'2), C(O)O(R'2), C(O)N(R'2)(R'3), C(=N(R'3)(R'2), C(=N(OR'3)) (R'2), S(R'2), S(O)(R'2), S(O₂)(R'2), S(O₂)O(R'2) und SO₂)N(R'2)(R'3); worin R'2, R'3, R'4 jeweils unabhängig ausgewählt ist aus der Gruppe bestehend aus H, Alkyl, Alkylen, Alkinyl, Aryl, Heteroaryl, Cycloalkyl, Heterocyclyl, substituiertem Alkyl, substituiertem Alkylen, substituiertem Alkinyl, substituiertem Aryl, substituiertem Heteroaryl, substituiertem Cycloalkyl und substituiertem Heterocyclyl und R'2 und R'3 unter Bildung eines Rings, der 1 bis 3 aus O, S und N ausgewählte Heteroatome enthält, miteinander verbunden sein können;
mit der Maßgabe, dass dann, wenn X für N steht, R3 für NH₂ steht, Ar und A für unsubstituiertes Phenyl stehen, L für NHCO, das in para-Stellung von Ar gebunden ist, steht und R5 für H steht, R4 nicht ausgewählt ist aus: Phenyl, *o*-Chlorphenyl, Cinnamyl, α-Furfuryl, *o*-Hydroxyphenyl, *p*-Hydroxym-methoxyphenyl, *p*-Methylthiophenyl, *p*-Methoxyphenyl, *o*-Nitrophenyl, *m*-Phenoxyphenyl, und mit der Maßgabe, dass dann, wenn X für N steht, R5 für H steht, R4 für H steht und Ar-L-A für eine Gruppe 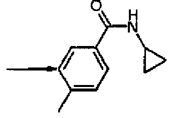 steht, R3 nicht ausgewählt ist aus: Amino, Acetylamino, [(4-Fluorphenyl)carbonyl]amino, (2-Methylpropanoyl)amino, -(Cyclopentylcarbonyl)-amino, Propanoylamino, [(4-Methylphenyl)carbonyl]-amino, {[4-(Methyloxy)phenyl]carbonyl}amino, (2-Thienylcarbonyl)amino, (Methylsulfonyl)amino, -(4-Fluorphenyl)sulfonyl]amino, (Ethylsulfonyl)amino, (Propylsulfanyl)amino, (3-Thienylsulfonyl)amino, [(3,5-Dimethyl-4-isoxazolyl)sulfonyl]amino, (2-Thienylsulfonyl)amino und (1-Methylethyl)amino.

2. Produkt nach Anspruch 1, **dadurch gekennzeichnet, dass**:
1) A und Ar unabhängig ausgewählt sind aus der Gruppe bestehend aus: Aryl, Heteroaryl, Heterocyclyl, Cycloalkyl, substituiertem Aryl, substituiertem Heteroaryl, substituiertem Heterocyclyl, und substituiertem Cycloalkyl;
2) L ausgewählt ist aus der Gruppe bestehend aus: NH, NH-SO₂, SO₂NH, NH-CH₂, CH₂-NH, CH₂-CO-NH, NH-CO-CH₂, NH-CH₂-CO, CO-CH₂-NH, NH-CO-NH, NH-CS-NH, NH-CO-O, O-CO-NH, CH₂-NH-CO-NH, NH-CO-NH-CH₂ und NH-CO-CH₂-CO-NH;
3) X für N steht;
4) R3 ausgewählt ist aus der Gruppe bestehend aus H, NH₂ und NHCOR''3, worin R''3 ausgewählt ist aus der Gruppe bestehend aus H, Alkyl, Alkylen, Alkinyl, Aryl, Heteroaryl, Cycloalkyl, Heterocyclyl, substituiertem Alkyl, substituiertem Alkylen, substituiertem Alkinyl, substituiertem Aryl, substituiertem Heteroaryl, substituiertem Cycloalkyl und substituiertem Heterocyclyl;
5) R4 ausgewählt ist aus der Gruppe bestehend aus: H, Halogen, Alkyl, substituiertem Alkyl, CON(R''5) (R"6), worin R"5 und R''6 unabhängig ausgewählt sind aus der Gruppe bestehend aus H, (C₁-C₆)Alkyl, substituiertem (C₁-C₆)Alkyl, -(C₁-C₆)Alkylheterocyclyl, substituiertem -(C₁-C₆)Alkylheterocyclyl, -(C₁-C₆)Alkylheteroaryl, substituiertem -(C₁-C₆)Alkylheteroaryl, Cycloalkyl, substituiertem Cycloalkyl, Heterocyclyl, substituiertem Heterocyclyl, Aryl, substituiertem Aryl, Heteroaryl und substituiertem Heteroaryl oder R''5 und R''6 unter Bildung eines 4- bis 8-gliedrigen gesättigten Rings, der 1 bis 3 aus O, S und N ausgewählte Heteroatome enthält und gegebenenfalls substituiert ist, miteinander verbunden sind;
6) R5 für H steht.

3. Produkt nach Anspruch 1 oder 2, **dadurch gekennzeichnet, dass** Ar ausgewählt ist aus der Gruppe bestehend aus: Thiazolyl, Thienyl, Furyl, Pyrrolyl, Oxazolyl, Isoxazolyl, Isothiazolyl, Thiadiazolyl, Pyrazolyl, Imidazolyl, Indolyl, Indazolyl, Benzimidazolyl, Benzoxazolyl und Benzothiazolyl; gegebenenfalls substituiert.

4. Produkt nach Anspruch 1 oder 2, **dadurch gekennzeichnet, dass** Ar-L-A für: 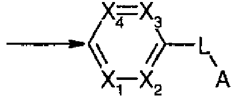
steht, worin X1, X2, X3 und X4 jeweils unabhängig aus N und C-R'5 ausgewählt sind, worin R'5 die gleiche Definition wie R5 hat.

5. Produkt nach Anspruch 1, **dadurch gekennzeichnet, dass** L-A aus der Gruppe bestehend aus NH-CO-NH-A und NH-SO₂-A ausgewählt ist.

6. Verfahren nach Anspruch 1, **dadurch gekennzeichnet, dass** A ausgewählt ist aus der Gruppe bestehend aus: Phenyl, Pyridyl, Pyrimidyl, Thienyl, Furyl, Pyrrolyl, Oxazolyl, Thiazolyl, Isoxazolyl, Isothiazolyl, Pyrazolyl, Imidazolyl, Indolyl, Indazolyl, Benzimidazolyl, Benzoxazolyl und Benzothiazolyl; gegebenenfalls substituiert.

7. Produkt nach Anspruch 6, **dadurch gekennzeichnet, dass** A ausgewählt ist aus der Gruppe bestehend aus: Phenyl, Pyrazolyl und Isoxazolyl; gegebenenfalls substituiert.

8. Produkt nach Anspruch 1, **dadurch gekennzeichnet, dass** A durch einen ersten Substituenten aus der Gruppe bestehend aus Halogen, Alkyl, Alkylen, Alkinyl, Aryl, Heteroaryl, O-Alkyl, O-Aryl, O-Heteroaryl, S-Alkyl, S-Aryl und S-Heteroaryl, jeweils gegebenenfalls substituiert durch einen oder mehrere aus (C₁-C₃)Alkyl, Halogen und O-(C₁C₃)Alkyl ausgewählte Substituenten, substituiert ist.

9. Produkt nach Anspruch 8, **dadurch gekennzeichnet, dass** A durch einen zweiten Substituenten aus der Gruppe bestehend aus F, Cl, Br, I, OH, SH, SO₃M, COOM, CN, NO₂, CON(R8)(R9), N(R8)CO(R9), (C₁-C₃) Alkyl-OH, (C₁-C₃) Alkyl-N (R8) (R9), (C₁-C₃) Alkyl-(R10), (C₁-C₃)Alkyl-COOH und N(R8) (R9) substituiert ist; worin R8 und R9 unabhängig ausgewählt sind aus H, (C₁-C₃)Alkyl, halogeniertem (C₁-C₃)Alkyl, (C₁-C₃)AlkylOH, (C₁-C₃)Alkyl-O (C₁-C₃) alkyl, (C₁-C₃)AlkylNH₂, (C₁-C₃)AlkylN(R8)(R9), (C₁-C₃)AlkylCOOM, (C₁-C₃)AlkylSO₃M; worin R8 und R9 dann, wenn sie gleichzeitig von H verschieden sind, unter Bildung eines 5- bis 7-gliedrigen Rings mit 1 bis 3 Heteroatomen verbunden sein können; worin M für H oder ein Kation eines aus Li, Na und K ausgewählten Alkalimetalls steht und worin R10 für H oder einen gegebenenfalls substituierten nichtaromatischen Heterocyclus mit 2 bis 7 Kohlenstoffatomen und 1 bis 3 aus N, O und S ausgewählten Heteroatomen steht.

10. Produkt nach Anspruch 1, **dadurch gekennzeichnet, dass** A ausgewählt ist aus Phenyl, Pyrazolyl und Isoxazolyl; gegebenenfalls substituiert durch Halogen, (C₁-C₄)Alkyl, halogeniertem (C₁-C₃)Alkyl, O-(C₁-C₄)Alkyl, S-(C₁-C₉)Alkyl, halogeniertem O-(C₁-C₄)Alkyl und halogeniertem S-(C₁-C₄)Alkyl, und dass dann, wenn A disubstituiert ist, die beiden Substituenten von A einen 5- bis 7-gliedrigen Ring mit 0 bis 3 aus O, N und S ausgewählten Heteroatomen bilden können.

11. Produkt nach Anspruch 1, **dadurch gekennzeichnet, dass** R4 für H oder CON (R''5)(R''6) steht, wobei R''5 und R''6 die oben angegebene Bedeutung besitzen.

12. Produkt nach einem der Ansprüche 1 bis 11, **dadurch gekennzeichnet, dass** es ausgewählt ist aus:
1-[4-(3-Amino-1H-pyrazolo[3,4-b]pyridin-6-yl)-phenyl]-3-(2-fluor-5-trifluormethylphenyl)harnstoff;
Thiophen-3-carbonsäure(5-{4-[3-(2-fluor-5-tri-fluormethylphenyl)ureido]phenyl}-1H-pyrazolo[3,4-b]pyridin-3-yl)amid;
5-{2-[3-(2-Fluor-5-trifluormethylphenyl)ureido]-thiazol-5-yl}-1H-pyrazolo[3,4-b]pyridin-4-carbonsäure(2-morpholin-4-ylethyl)amid-trifluoracetat;
6-{4-[3-(2-Fluor-5-trifluormethylphenyl)ureido]-phenyl}-1H-pyrazolo[3,4-b]pyridin-4-carbonsäureamid;
6-{4-[3-(2-Fluor-5-trifluormethylphenyl)ureido]-phenyl}-1H-pyrazolo[3,4-b]pyridin-4-carbonsäuremethylamid;
6-{4-[3-(2-Fluor-5-trifluormethylphenyl)ureido]-phenyl}-1H-pyrazolo[3,4-b]pyridin-4-carbonsäure(2-morpholin-9-ylethyl)amid;
6-[2-(3-Phenylureido)thiazol-5-yl]-1H-pyrazolo-[3,4-b]pyridin-4-carbonsäure(2-morpholin-4-yl-ethyl)amid;
6-{2-[3-(2-Fluor-5-trifluormethylphenyl)ureido]-thiazol-5-yl}-1H-pyrazolo[3,4-b]pyridin-4-carbon-säureamid;
6-{2-[3-(2-Fluor-5-trifluormethylphenyl)ureido]-thiazol-5-yl}-1H-pyrazolo[3,4-b]pyridin-4-carbon-säuremethylamid;
6-{2-[3-(2-Fluor-5-trifluormethylphenyl)ureido]-thiazol-5-yl}-1H-pyrazolo[3,4-b]pyridin-4-carbon-säure(2-dimethylaminoethyl)amid;
6-{2-[3-(2-Fluor-5-trifluormethylphenyl)ureido]-thiazol-5-yl}-1H-pyrazolo[3,4-b]pyridin-4-carbonsäure(3-dimethylamino-2,2-dimethylpropyl)amid;
6-{2-[3-(2-Fluor-5-trifluormethylphenyl)ureido]-thiazol-5-yl}-1H-pyrazolo[3,4-b]pyridin-4-carbonsäure[2-(3H-imidazol-4-yl)ethyl]amid;
1-((2-Fluor-5-trifluormethylphenyl)-3-{5-[4-(morpholin-4-carbonyl)-1H-pyrazolo[3,4-b]pyridin-6-yl]thiazol-2-yl}harnstoff;
1-((2-Fluor-5-trifluormethylphenyl)-3-{5-[4-(piperazin-1-carbonyl)-1H-pyrazolo[3,4-b]pyridin-6-yl]-thiazol-2-yl}harnstoff;
1-((2-Fluor-5-trifluormethylphenyl)-3-{5-[4-(4-methylpiperazin-1-carbonyl)-1H-pyrazolo[3,4-b]-pyridin-6-yl]-thiazol-2-yl}harnstaff;
6-{2-[3-(2-Fluor-5-trifluormethylphenyl)ureido]-thiazol-5-yl}-1H-pyrazolo[3,4-b]pyridin-4-carbon-säure(2,3-dihydroxypropyl)amid;
6-{2-[3-(2-Fluor-5-trifluormethylphenyl)ureido]-thiazol-5-yl}-1H-pyrazolo[3,4-b]pyridin-4-carbon-säure(2H-pyrazol-3-yl)amid;
6-{6-[3-(2-Fluor-5-trifluormethylphenyl)ureido]-pyridin-3-yl}-1H-pyrazolo[3,4-b]pyridin-4-carbon-säuremethylamid;
6-{6-[3-(2-Fluor-5-trifluormethylphenyl)ureido]-pyridin-3-yl}-1H-pyrazolo[3,4-b]pyridin-4-carbon-säure(2-morpholin-4-ylethyl)amid;
6-{2-[3-(2-Fluor-5-trifluormethylphenyl)ureido]-thiazol-5-yl}-1H-pyrazolo[3,4-b]pyridin-4-carbon-säure(3-morpholin-4-ylpropyl)amid;
6-{2-[3-(2-Fluor-5-trifluormethylphenyl)ureido]-thiazol-5-yl}-1H-pyrazolo[3,4-b]pyridin-4-carbon-säure(2-piperazin-1-ylethyl)amid;
6-{2-[3-(2-Fluor-5-trifluormethylphenyl)ureido]-thiazol-5-yl}-1H-pyrazolo[3,4-b]pyridin-4-carbonsäure(2-piperidin-4-ylethyl)amid;
6-{2-[3-(2-Fluor-5-trifluormethylphenyl)ureido]-thiazol-5-yl}-1H-pyrazolo[3,4-b]pyridin-4-carbonsäure[3-(4-methylpiperazin-1-yl)propyl]amid;
6-{2-[3-(2-Fluor-5-trifluormethylphenyl)ureido]-thiazol-5-yl}-1H-pyrazolo[3,4-b]pyridin-4-carbon-säure(2-hydroxyethyl)amid;
6-{2-[3-(2-Fluor-5-trifluormethylphenyl)ureido]-thiazol-5-yl}-1H-pyrazolo[3,4-b]pyridin-4-carbon-säure(2-methoxyethyl)amid;
6-{2-[3-(2-Fluor-5-trifluormethylphenyl)ureido]-thiazol-5-yl}-1H-pyrazolo[3,4-b]pyridin-4-carbon-säure(pyridin-4-ylmethyl)amid;
6-{2-[3-(2-Fluor-5-trifluormethylphenyl)ureido]-thiazol-5-yl}-1H-pyrazolo[3,4-b]pyridin-4-carbon-säure(pyridin-2-ylmethyl)amid;
1-((2-Fluor-5-trifluormethylphenyl)-3-{5-[4-(2-hydroxymethylpyrrolidin-1-carbonyl)-1H-pyrazolo-[3,4-b]pyridin-6-yl]thiazol-2-yl}harnstoff;
6-{2-[3-(2-Fluor-5-trifluormethylphenyl)ureido]-thiazol-5-yl}-1H-pyrazolo[3,4-b]pyridin-4-carbon-säure[3-(2-hydroxymethylpyrrolidin-1-yl)propyl]-amid;
1-((2-Fluor-5-trifluormethylphenyl)-3-{5-[4-(2-hydroxymethylpiperazin-1-carbonyl)-1H-pyrazolo-[3,4-b]pyridin-6-yl]thiazol-2-yl}harnstoff;
6-{3-[3-(2-Fluor-5-trifluormethylphenyl)ureido]-phenyl}-1H-pyrazolo[3,4-b]pyridin-4-carbonsäure-methylamid;
6-{3-[3-(2-Fluor-5-trifluormethylphenyl)ureido]-phenyl}-1H-pyrazolo[3,4-b]pyridin-4-carbonsäure(2-morpholin-4-ylethyl)amid;
6-{5-[3-(2-Fluor-5-trifluormethylphenyl)ureido]-isoxazol-3-yl}-1H-pyrazolo[3,4-b]pyridin-4-carbonsäure(2-morpholin-4-ylethyl)amid;
6-{2-[3-(2-Fluor-5-trifluormethylphenyl)ureido]-thiazol-5-y1}-1H-pyrazolo[3,4-b]pyridin-4-carbon-säure(pyridin-3-ylmethyl)amid;
6-{2-[3-(2-Fluor-5-trifluormethylphenyl)ureido]-thiazol-5-yl}-1H-pyrazolo[3,4-b]pyridin-4-carbon-säure[2-(4-methylpiperazin-1-yl)ethyl]amid;
6-{5-[3-(2-Fluor-5-trifluormethylphenyl)ureido]-[1,3,4]thiadiazol-2-yl}-1H-pyrazolo[3,4-b]pyridin-9-carbonsäure(2-morpholin-4-ylethyl)amid;
6-{5-[3-(2-Fluor-5-trifluormethylphenyl)ureido]-thiophen-2-yl}-1H-pyrazolo[3,4-b]pyridin-4-carbon-säure(2-morpholin-9-ylethyl)amid;
6-{2-[3-(3-Trifluormethylphenyl)ureido]thiazol-5-yl}-1H-pyrazolo[3,4-b]pyridin-4-carbonsäure(2-morpholin-4-ylethyl)amid;
6-{2-[3-(3-Trifluormethylsulfanylphenyl)ureido]-thiazol-5-yl}-1H-pyrazolo[3,4-b]pyridin-4-carbonsäure(2-morpholin-4-ylethyl)amid;
6-{2-[3-(3-Fluor-5-trifluormethylphenyl)ureido]-thiazol-5-yl}-1H-pyrazolo[3,4-b]pyridin-4-carbonsäure(2-morpholin-4-ylethyl)amid;
6-{2-[3-(4-Fluor-3-trifluormethylphenyl)ureido]-thiazol-5-yl}-1H-pyrazolo[3,4-b]pyridin-4-carbon-säure(2-morpholin-4-ylethyl)amid;
6-{2-[3-(2-Chlorphenyl)ureido]thiazol-5-yl}-1H-pyrazolo[3,4-b]pyridin-4-carbonsäure(2-morpholin-4-ylethyl)amid;
6-{2-[3-(3-Chlorphenyl)ureido]thiazol-5-yl}-1H-pyrazolo[3,4-b]pyridin-4-carbonsäure(2-morpholin-4-ylethyl)amid;
6-{2-[3-(2-Fluor-3-trifluormethylphenyl)ureido]-thiazol-5-yl}-1H-pyrazolo[3,4-b]pyridin-4-carbonsäure(2-morpholin-4-ylethyl)amid;
6-{2-[3-(3-Chlor-4-fluorphenyl)ureido]thiazol-5-yl}-1H-pyrazolo[3,4-b]pyridin-4-carbonsäure(2-morpholin-4-ylethyl)amid;
6-{2-[3-(2-Methoxyphenyl)ureido]thiazol-5-yl}-1H-pyrazolo[3,4-b]pyridin-4-carbonsäure(2-morpholin-4-ylethyl)amid;
6-{2-[3-(2,5-Difluorphenyl)ureido]thiazol-5-yl}-1H-pyrazolo[3,4-b]pyridin-4-carbonsäure(2-morpholin-4-ylethyl)amid;
6-{2-[3-(2,4-Difluorphenyl)ureido]thiazol-5-yl}-1H-pyrazolo[3,4-b]pyridin-4-carbonsäure(2-morpholin-4-ylethyl)amid;
6-{2-[3-(4-Trifluormethylphenyl)ureido]thiazol-5-yl}-1H-pyrazolo[3,4-b]pyridin-4-carbonsäure(2-morpholin-4-ylethyl)amid;
6-{2-[3-(2-Fluorphenyl)ureido]thiazol-5-yl}-1H-pyrazolo[3,4-b]pyridin-4-carbonsäure(2-morpholin-4-ylethyl)amid;
6-{2-[3-(3,4-Dichlorphenyl)ureido]thiazol-5-yl}-1H-pyrazolo[3,4-b]pyridin-4-carbonsäure(2-morpholin-4-ylethyl)amid;
6-{2-[3-(4-Trifluormethoxyphenyl)ureido]thiazol-5-yl}-1H-pyrazolo[3,4-b]pyridin-4-carbonsäure(2-morpholin-4-ylethyl)amid;
6-{2-[3-(3-Cyanophenyl)ureido]thiazol-5-yl}-1H-pyrazalo[3,4-b]pyridin-4-carbonsäure(2-morphoiin-4-ylethyl)amid;
6-{2-[3-(3-Methoxyphenyl)ureido]thiazol-5-yl}-1H-pyrazolo[3,4-b]pyridin-4-carbonsäure(2-morpholin-4-ylethyl)amid:
6-{2-[3-(4-Chlorphenyl)ureido]thiazol-5-yl}-1H-pyrazolo[3,4-b]pyridin-4-carbonsäure(2-morpholin-4-ylethyl)amid;
6-{2-[3-(2,4-Dimethoxyphenyl)ureido]thiazol-5-yl}-1H-pyrazolo[3,4-b]pyridin-4-carbonsäure(2-morpholin-4-ylethyl)amid.

13. Produkt nach einem der Ansprüche 1 bis 11, **dadurch gekennzeichnet, dass** es ausgewählt ist aus:
N-[4-(4-(2-Morpholin-4-ylethyl)aminocarbonyl-1H-pyrazolo[3,4-b]pyridin-6-yl)phenyl]-3-chlorbenzol-sulfonamid;
N-[4-(4-(Piperazin-1-carbanyl)-1H-pyrazolo[3,4-b]-pyridin-6-yl)phenyl]-2,3-dichlorbenzolsulfonamid;
N-[4-(4-Methylaminocarbonyl-1H-pyrazolo[3,4-b]-pyridin-6-yl)phenyl]-2-chlor-4-trifluormethyl-benzolsulfonamid;
N-[4-(4-Methylaminocarbonyl-1H-pyrazolo[3,9-b]-pyridin-6-yl)phenyl]-4-fluorbenzolsulfonamid;
N-[4-(3-Amino-1H-pyrazolo[3,4-b]pyridin-6-yl)-phenyl]-2,3-dichlorbenzolsulfonamid;
N-[4-(3-Amino-4-methylaminocarbonyl-1H-pyrazolo-[3,4-b]pyridin-6-yl)phenyl]-2,3-dichlorbenzol-sulfonamid.

14. Produkt nach einem der vorhergehenden Ansprüche, **dadurch gekennzeichnet, dass** es
a) in achiraler Form oder
b) in racemischer Form oder
c) in mit einem Stereoisomer angereicherter Form oder
d) in mit einem Enantiomer angereicherter Form vorliegt und gegebenenfalls versalzt ist.

15. Medikament, **dadurch gekennzeichnet, dass** es ein Produkt der Formel (I) nach einem der Ansprüche 1 bis 14 oder ein Additionssalz dieses Produkts mit einer pharmazeutisch unbedenklichen Säure oder auch ein Hydrat oder ein Solvat des Produkts der Formel (I) enthält.

16. Pharmazeutische Zusammensetzung, umfassend ein Produkt nach einem der vorhergehenden Ansprüche in Kombination mit mindestens einem pharmazeutisch unbedenklichen Träger.

17. Produkt nach einem der Ansprüche 1 bis 14 zur Verwendung als Mittel zur Inhibierung einer durch eine oder mehrere Kinasen katalysierten Reaktion.

18. Produkt zur Verwendung nach Anspruch 17, **dadurch gekennzeichnet, dass** die Kinase aus FAK, KDR und Tie2 ausgewählt ist.

19. Verwendung eines Produkts nach einem der Ansprüche 1 bis 14 zur Herstellung eines Medikaments zur Behandlung eines pathologischen Zustands.

20. Verwendung nach Anspruch 19, **dadurch gekennzeichnet, dass** es sich bei dem pathologischen Zustand um Krebs handelt.

21. Verwendung nach Anspruch 19 oder 20, **dadurch gekennzeichnet, dass** der pathologische Zustand ausgewählt ist aus rheumatoider Arthritis, Osteoarthritis und/oder damit assoziierten Schmerzen, entzündlichen Darmerkrankungen wie Colitis ulcerosa oder Morbus Crohn, Pathologien des Auges wie altersbedingter Makuladegeneration, diabetischen Retinopathien, chronischer Entzündung, Psoriasis und speziellen Krebserkrankungen wie Kaposi-Sarkom oder infantilem Hämangiom.

22. Verwendung nach den Ansprüchen 19 bis 21 eines Produkts nach einem der Ansprüche 1 bis 14 zur Behandlung oder Prävention eines pathologischen Zustands, **dadurch gekennzeichnet, dass** das Produkt alleine oder in Kombination mit anderen Wirkstoffen, insbesondere Antikrebsmitteln wie zytotoxischen, zytostatischen, antiangiogenen oder antimetastatischen Mitteln, verabreicht wird.

## Claims

1. Product of general formula (I) below: 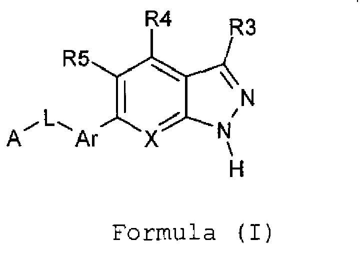 in which:
1) A and Ar are independently selected from the group consisting of: aryl, heteroaryl, heterocyclyl, substituted aryl, substituted heteroaryl, substituted heterocyclyl, cycloalkyl and substituted cycloalkyl;
2) L is selected from the group consisting of: NH, NH-SO₂, SO₂NH, NH-CH₂, CH₂-NH, NH-CO, CO-NH, CH₂-CO-NH, NH-CO-CH₂, NH-CH₂-CO, CO-CH₂-NH, NH-CO-NH, NH-CS-NH, NH-CO-O, O-CO-NH, CH₂-NH-CO-NH, NH-CO-NH-CH₂, and NH-CO-CH₂-CO-NH;
3) X is N or NO;
4) R3 is selected from the group consisting of H and NHMR"3, in which M is selected from the group consisting of: a bond, CO, CO-NH, CS, CS-NH and SO_{2;} and in which R"3 is selected from the group consisting of H, alkyl, alkylene, alkynyl, aryl, heteroaryl, cycloalkyl, heterocyclyl, substituted alkyl, substituted alkylene, substituted alkynyl, substituted aryl, substituted heteroaryl, substituted cycloalkyl and substituted heterocyclyl;
5) R4 is selected from the group consisting of: H, halogen, alkyl, substituted alkyl, OR"4, N(R"5) (R"6), CON(R"5) (R"6), in which R"4 is chosen from H, phenyl, substituted phenyl, alkyl, substituted alkyl, and in which R"5 and R"6 are independently selected from the group consisting of H, (C₁-C₆)alkyl, substituted (C₁-C₆)alkyl, - (C₁-C₆)alkylheterocyclyl, substituted -(C₁-C₆)alkylheterocyclyl, -(C₁-C₆)alkylheteroaryl, substituted -(C₁-C₆)alkylheteroaryl, cycloalkyl, substituted cycloalkyl, heterocyclyl, substituted heterocyclyl, aryl, substituted aryl, heteroaryl, substituted heteroaryl, or else R"5 and R"6 are linked to one another so as to form a saturated ring having from 4 to 8 ring members containing from 1 to 3 hetero atoms chosen from 0, S and N, optionally substituted;
6) R5 is selected from the group consisting of: H, halogen, R'2, CN, O(R'2), OC(O)(R'2), OC(O)N(R'2) (R'3), OS(O₂)(R'2), N(R'2) (R'3), N=C(R'2)(R'3), N(R'2)C(O)(R'3), N(R'2)C(O)O(R'3), N(R'4)C(O)N(R'2)(R'3), N(R'4)C(S)N(R'2)(R'3), N(R'2)S(O₂) (R'3), C(O)(R'2), C(O)O(R'2), C(O)N(R'2)(R'3), C(=N(R'3))(R'2), C(=N(OR'3)) (R'2), S(R'2), S(O)(R'2), S(O2)(R'2), S(2)O(R'2), S(O₂)N(R'2)(R'3); in which each R'2, R'3, R'4 is independently selected from the group consisting of H, alkyl, alkylene, alkynyl, aryl, heteroaryl, cycloalkyl, heterocyclyl, substituted alkyl, substituted alkylene, substituted alkynyl, substituted aryl, substituted heteroaryl, substituted cycloalkyl, substituted heterocyclyl; and R'2 and R'3 can be linked to one another so as to form a ring containing from 1 to 3 hetero atoms chosen from O, S and N;
provided that, when X is N, R3 is NH₂, Ar and A are unsubstituted phenyl, L is NHCO linked in the para-position with respect to Ar, and R5 is H, then R4 is not chosen from: phenyl, O-chlorophenyl, cinnamyl, α-furfuryl, O-hydroxyphenyl, p-hydroxy-m-methoxyphenyl, p-methylthiophenyl, p-methoxyphenyl, o-nitrophenyl, m-phenoxyphenyl, and provided that, when X is N, R5 is H, R4 is H, and Ar-L-A is a group 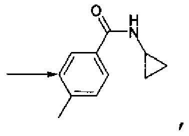 then R3 is not chosen from: amino, acetylamino, [(4-fluorophenyl)carbonyl]amino, (2-methylpropanoyl)amino, -(cyclopentylcarbonyl)amino, propanoylamino, [(4-methylphenyl)carbonyl]amino, {[4-(methyloxy)phenyl]carbonyl}amino, (2-thienylcarbonyl)amino, (methylsulphonyl)amino, - [(4-fluorophenyl)sulphonyl]amino, (ethylsulphonyl)amino, (propylsulphonyl) amino, (3-thienylsulphonyl)amino, [(3,5-dimethyl-4-isoxazolyl)sulphonyl]amino, (2-thienylsulphonyl)amino and (1-methylethyl)amino.

2. Product according to Claim 1, **characterized in that**:
1) A and Ar are independently selected from the group consisting of: aryl, heteroaryl, heterocyclyl, cycloalkyl, substituted aryl, substituted heteroaryl, substituted heterocyclyl and substituted cycloalkyl;
2) L is selected from the group consisting of: NH, NH-SO₂, SPO₂NH, NH-CH₂, CH₂-NH, CH₂-CO-NH, NH-CO-CH₂, NH-CH₂-CO, CO-CH₂-NH, NH-CO-NH, NH-CS-NH, NH-CO-O, O-CO-NH, CH₂-NH-CO-NH, NH-CO-NH-CH₂ and NH-CO-CH₂-CO-NH;
3) X is N;
4) R3 is selected from H, NH2 and NHCOR"3, in which R" 3 is selected from the group consisting of H, alkyl, alkylene, alkynyl, aryl, heteroaryl, cycloalkyl, heterocyclyl, substituted alkyl, substituted alkylene, substituted alkynyl, substituted aryl, substituted heteroaryl, substituted cycloalkyl and substituted heterocyclyl;
5) R4 is selected from the group consisting of: H, halogen, alkyl, substituted alkyl, CON(R''5)(R''6) in which R"5 and R"6 are independently selected from the group consisting of H, (C₁-C₆)alkyl, substituted (C₁-C₆) alkyl, -(C₁-C₆)alkylheterocyclyl, substituted - (C₁-C₆) alkylheterocyclyl, - (C₁-C₆)alkylheteroaryl, substituted -(C₁-C₆)alkylheteroaryl, cycloalkyl, substituted cycloalkyl, heterocyclyl, substituted heterocyclyl, aryl, substituted aryl, heteroaryl, substituted heteroaryl or else R"5 and R"6 are linked to one another so as to form a saturated ring having from 4 to 8 ring members containing from 1 to 3 hetero atoms chosen from O, S and N, optionally substituted;
6) R5 is H.

3. Product according to Claim 1 or 2, **characterized in that** Ar is chosen from the group consisting of: thiazolyl, thienyl, furyl, pyrrolyl, oxazolyl, isoxazolyl, isothiazolyl, thiadiazolyl, pyrazolyl, imidazolyl, indolyl, indazolyl, benzimidazolyl, benzoxazolyl and benzothiazolyl; optionally substituted.

4. Product according to Claim 1 or 2, **characterized in that** Ar-L-A is: 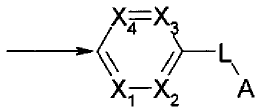
in which each X1, X2, X3 and X4 is independently chosen from N and C-R'5, in which R'5 has the same definition as R5.

5. Product according to Claim 1, **characterized in that** L-A is selected from the group consisting of NH-CO-NH-A and NH-SO₂-A.

6. Product according to Claim 1, **characterized in that** A is selected from the group consisting of: phenyl, pyridyl, pyrimidyl, thienyl, furyl, pyrrolyl, oxazolyl, thiazolyl, isoxazolyl, isothiazolyl, pyrazolyl, imidazolyl, indolyl, indazolyl, benzimidazolyl, benzoxazolyl and benzothiazolyl; optionally substituted.

7. Product according to Claim 6, **characterized in that** A is selected from the group consisting of: phenyl, pyrazolyl and isoxazolyl; optionally substituted.

8. Product according to Claim 1, **characterized in that** A is substituted with a first substituent selected from the group consisting of halogen, alkyl, alkylene, alkynyl, aryl, heteroaryl, O-alkyl, 0-aryl, O-heteroaryl, S-alkyl, S-aryl, S-heteroaryl, each being optionally substituted with one or more substituents chosen from (C₁-C₃)alkyl, halogen and O-(C₁-C₃)alkyl.

9. Product according to Claim 8, **characterized in that** A is substituted with a second substituent selected from the group consisting of F, Cl, Br, I, OH, SH, SO₃M, COOM, CN, NO₂, CON(R8)(R9), N(R8)CO(R9), (C₁-C₃)alkyl-OH, (C₁-C₃) alkyl-N(R8)(R9), (C₁-C₃) alkyl-(R10), (C₁-C₃)alkyl-COOH, N(R8)(R9); in which R8 and R9 are independently chosen from H, (C₁-C₃) alkyl, halogenated (C₁-C₃)alkyl, (C₁-C₃)alkylOH, (C₁-C₃)alkyl-O(C₁-C₃) alkyl, (C₁-C₃) alkylNH₂, (C₁-C₃) alkyl (R8) (R9), (C₁-C₃)alkylCOOM, (C₁-C₃) alkylSO₃M; in which, when R8 and R9 are simultaneously different from H, they can be linked so as to form a ring having from 5 to 7 ring members containing from 1 to 3 hetero atoms; in which M is H or a cation of an alkali metal chosen from Li, Na and K; and in which R10 is H or an optionally substituted, non-aromatic heterocycle containing 2 to 7 carbon atoms and 1 to 3 hetero atoms chosen from N, O and S.

10. Product according to Claim 1, **characterized in that** A is chosen from phenyl, pyrazolyl and isoxazolyl; optionally substituted with halogen, (C₁-C₉)alkyl, halogenated (C₁-C₃)alkyl, O-(C₁-C₉)alkyl, S- (C₁-C₄alkyl, halogenated O-(C₁-C₄alkyl, and halogenated S-(C₁-C₄)alkyl, and **in that**, when A is disubstituted, the two substituents of A can form a ring having from 5 to 7 ring members containing from 0 to 3 hetero atoms chosen from O, N and S.

11. Product according to Claim 1, **characterized in that** R4 is H or CON(R"5)(R"6), with R" 5 and R" 6 as defined above.

12. Product according to any one of Claims 1 to 11, **characterized in that** it is chosen from:
1-[4-(3-amino-1H-pyrazolo[3,4-b]pyridin-6-yl)phenyl]-3-(2-fluoro-5-trifluoromethylphenyl)urea;
thiophene-3-carboxylic acid (6-{4-[3-(2-fluoro-5-trifluoromethylphenyl)ureido]phenyl}-1H-pyrazolo[3,4-b]pyridin-3-yl)amide;
6-{2-[3-(2-fluoro-5-trifluoromethylphenyl)ureido]thiazol-5-yl}-1H-pyrazolo[3,4-b]pyridine-4-carboxylic acid (2-morpholin-4-ylethyl)amide trifluoroacetate;
6-{4-[3-(2-fluoro-5-trifluoromethylphenyl)ureido]phenyl}-1H-pyrazolo[3,4-b]pyridine-4-carboxylic acid amide;
6-{4-[3-(2-fluoro-5-trifluoromethylphenyl)ureido]phenyl}-1H-pyrazolo[3,4-b]pyridine-4-carboxylic acid methylamide;
6-{4-[3-(2-fluoro-5-trifluoromethylphenyl)ureido]phenyl}-1H-pyrazolo[3,4-b]pyridine-4-carboxylic acid (2-morpholin-4-ylethyl)amide;
6-[2-(3-phenylureido)thiazol-5-yl]-1H-pyrazolo[3,4-b]pyridine-4-carboxylic acid (2-morpholin-4-ylethyl)amide;
6-{2-[3-(2-fluoro-5-trifluoromethylphenyl)ureido]thiazol-5-yl}-1H-pyrazolo[3,4-b]pyridine-4-carboxylic acid amide;
6-{2-[3-(2-fluoro-5-trifluoromethylphenyl)ureido]thiazol-5-yl}-1H-pyrazolo[3,4-b]pyridine-4-carboxylic acid methylamide;
6-{2-[3-(2-fluoro-5-trifluoromethylphenyl)ureido]thiazol-5-yl}-1H-pyrazolo[3,4-b]pyridine-4-carboxylic acid (2-dimethylaminoethyl)amide:
6-{2-[3-(2-fluoro-5-trifluoromethylphenyl)ureido]thiazol-5-yl}-1H-pyrazolo[3,4-b]pyridine-4-carboxylic acid (3-dimethylamino-2,2-dimethylpropyl)amide;
6-{2-[3-(2-fluoro-5-trifluoromethylphenyl)ureido]thiazol-5-yl}-1H-pyrazolo[3,4-b]pyridine-4-carboxylic acid [2-(3H-imidazol-4-yl)ethyl]amide;
1-((2-fluoro-5-trifluoromethylphenyl)-3-{5-[4-(morpholine-4-carbonyl)-1H-pyrazolo[3,4-b]pyridin-6-yl]thiazol-2-yl}urea;
1-((2-fluoro-5-trifluoromethylphenyl)-3-{5-[4-(piperazine-1-carbonyl)-1H-pyrazolo[3,4-b]pyridin-6-yl]-thiazol-2-yl}urea;
1-((2-fluoro-5-trifluoromethylphenyl)-3-{5-[4-(4-methylpiperazine-1-carbonyl)-1H-pyrazolo[3,4-b]pyridin-6-yl]-thiazol-2-yl}urea;
6-{2-[3-(2-fluoro-5-trifluoromethylphenyl)ureido]thiazol-5-yl}-1H-pyrazolo[3,4-b]pyridine-4-carboxylic acid (2,3-dihydroxypropyl)amide;
6-{2-[3-(2-fluoro-5-trifluoromethylphenyl)ureido]thiazol-5-yl}-1H-pyrazolo[3,4-b]pyridine-4-carboxylic acid (2H-pyrazol-3-yl)amide;
6-{6-[3-(2-fluoro-5-trifluoromethylphenyl)ureido]pyridin-3-yl}-1H-pyrazolo[3,4-b]pyridine-4-carboxylic acid methylamide;
6-{6-[3-(2-fluoro-5-trifluoromethylphenyl)ureido]pyridin-3-yl}-1H-pyrazolo[3,4-b]pyridine-4-carboxylic acid (2-morpholin-4-ylethyl)amide;
6-{2-[3-(2-fluoro-5-trifluoromethylphenyl)ureido]thiazol-5-yl}-1H-pyrazolo[3,4-b]pyridine-4-carboxylic acid (3-morpholin-4-ylpropyl)amide;
6-{2-[3-(2-fluoro-5-trifluoromethylphenyl)ureido]thiazol-5-yl}-1H-pyrazolo[3,4-b]pyridine-4-carboxylic acid (2-piperazin-1-ylethyl)amide;
6-{2-[3-(2-fluoro-5-trifluoromethylphenyl)ureido]thiazol-5-yl}-1H-pyrazolo[3,4-b]pyridine-4-carboxylic acid (2-piperidin-4-ylethyl)amide;
6-{2-[3-(2-fluoro-5-trifluoromethylphenyl)ureido]thiazol-5-yl}-1H-pyrazolo[3,4-b]pyridine-4-carboxylic acid [3-(4-methylpiperazin-1-yl)propyl]amide;
6-{2-[3-(2-fluoro-5-trifluoromethylphenyl)ureido]thiazol-5-yl}-1H-pyrazolo[3,4-b]pyridine-4-carboxylic acid (2-hydroxyethyl)amide;
6-{2-[3-(2-fluoro-5-trifluoromethylphenyl)ureido]thiazol-5-yl}-1H-pyrazolo[3,4-b]pyridine-4-carboxylic acid (2-methoxyethyl)amide;
6-{2-[3-(2-fluoro-5-trifluoromethylphenyl)ureido]thiazol-5-yl}-1H-pyrazolo[3,4-b]pyridine-4-carboxylic acid (pyridin-4-ylmethyl)amide;
6-{2-[3-(2-fluoro-5-trifluoromethylphenyl)ureido]thiazol-5-yl}-1H-pyrazolo[3,4-b]pyridine-4-carboxylic acid (pyridin-2-ylmethyl)amide;
1-((2-fluoro-5-trifluoromethylphenyl)-3-{5-[4-(2-hydroxymethylpyrrolidine-1-carbonyl)-1H-pyrazolo[3,4-b]pyridin-6-yl]thiazol-2-yl}urea;
6-{2-[3-(2-fluoro-5-trifluoromethylphenyl)ureido]thiazol-5-yl}-1H-pyrazolo[3,4-b]pyridine-4-carboxylic acid [3-(2-hydroxymethylpyrrolidin-1-yl)propyl]amide;
1-((2-fluoro-5-trifluoromethylphenyl)-3-{5-[4-(2-hydroxymethylpiperazine-1-carbonyl)-1H-pyrazolo[3,4-b]pyridin-6-yl]thiazol-2-yl}urea;
6-{3-[3-(2-fluoro-5-trifluoromethylphenyl)ureido]phenyl}-1H-pyrazolo[3,4-b]pyridine-4-carboxylic acid methylamide;
6-{3-[3-(2-fluoro-5-trifluoromethylphenyl)ureido]phenyl}-1H-pyrazolo[3,4-b]pyridine-4-carboxylic acid (2-morpholin-4-ylethyl)amide;
6-{5-[3-(2-fluoro-5-trifluoromethylphenyl)ureido]isoxazol-3-yl}-1H-pyrazolo[3,4-b]pyridine-4-carboxylic acid (2-morpholin-4-ylethyl)amide;
6-{2-[3-(2-fluoro-5-trifluoromethylphenyl)ureido]thiazol-5-yl}-1H-pyrazolo[3,4-b]pyridine-4-carboxylic acid (pyridin-3-ylmethyl)amide;
6-{2-[3-(2-fluoro-5-trifluoromethylphenyl)ureido]thiazol-5-yl}-1H-pyrazolo[3,4-b]pyridine-4-carboxylic acid [2-(4-methylpiperazin-1-yl)ethyl]amide;
6-{5-[3-(2-fluoro-5-trifluoromethylphenyl)ureido][1,3,4]thiadiazol-2-yl}1H-pyrazolo[3,4-b]pyridine-4-carboxylic acid (2-morpholin-4-ylethyl)amide;
6-{5-[3-(2-fluoro-5-trifluoromethylphenyl)ureido]thiophen-2-yl}-1H-pyrazolo[3,4-b]pyridine-4-carboxylic acid (2-morpholin-4-ylethyl)amide;
6-{2-[3-(3-trifluoromethylphenyl)ureido]thiazol-5-yl}-1H-pyrazolo[3,4-b]pyridine-4-carboxylic acid (2-morpholin-4-ylethyl)amide;
6-{2-[3-(3-trifluoromethylsulphanylphenyl)ureido]thiazol-5-yl}-1H-pyrazolo[3,4-b]pyridine-4-carboxylic acid (2-morpholin-4-ylethyl)amide;
6-{2-[3-(3-fluoro-5-trifluoromethylphenyl)ureido]thiazol-5-yl]-1H-pyrazolo[3,4-b]pyridine-4-carboxylic acid (2-morpholin-4-ylethyl)amide;
6-{2-[3-(4-fluoro-3-trifluoromethylphenyl)ureido]thiazol-5-yl}-1H-pyrazolo[3,4-b]pyridine-4-carboxylic acid (2-morpholin-4-ylethyl)amide;
6-{2-[3-(2-chlorophenyl)ureido]thiazol-5-yl}-1H-pyrazolo[3,4-b]pyridine-4-carboxylic acid (2-morpholin-4-ylethyl)amide;
6-{2-[3-(3-chlorophenyl)ureido]thiazol-5-yl}-1H-pyrazolo[3,4-b]pyridine-4-carboxylic acid (2-morpholin-4-ylethyl)amide;
6-{2-[3-(2-fluoro-3-trifluoromethylphenyl)ureido]thiazol-5-yl}-1H-pyrazolo[3,4-b]pyridine-4-carboxylic acid (2-morpholin-4-ylethyl)amide;
6-{2-[3-(3-chloro-4-fluorophenyl)ureido]thiazol-5-yl}-1H-pyrazolo[3,4-b]pyridine-4-carboxylic acid (2-morpholin-4-ylethyl)amide;
6-{2-[3-(2-methoxyphenyl)ureido]thiazol-5-yl}-1H-pyrazolo[3,4-b]pyridine-4-carboxylic acid (2-morpholin-4-ylethyl)amide;
6-{2-[3-(2,5-difluorophenyl)ureido]thiazol-5-yl}-1H-pyrazolo[3,4-b]pyridine-4-carboxylic acid (2-morpholin-4-ylethyl)amide;
6-{2-[3-(2,4-difluorophenyl)ureido]thiazol-5-yl}-1H-pyrazolo[3,4-b]pyridine-4-carboxylic acid (2-morpholin-4-ylethyl)amide;
6-{2-[3-(4-trifluoromethylphenyl)ureido]thiazol-5-yl}-1H-pyrazolo[3,4-b]pyridine-4-carboxylic acid (2-morpholin-4-ylethyl)amide;
6-{2-[3-(2-fluorophenyl)ureido]thiazol-5-yl}-1H-pyrazolo[3,4-b]pyridine-4-carboxylic acid (2-morpholin-4-ylethyl)amide;
6-{2-[3-(3,4-dichlorophenyl)ureido]thiazol-5-yl}-1H-pyrazolo[3,4-b]pyridine-4-carboxylic acid (2-morpholin-4-ylethyl)amide;
6-{2-[3-(4-trifluoromethoxyphenyl)ureido]thiazol-5-yl}-1H-pyrazolo[3,4-b]pyridine-4-carboxylic acid (2-morpholin-4-ylethyl)amide;
5-{2-[3-(3-cyanophenyl)ureido]thiazol-5-yl}-1H-pyrazolo[3,4-b]pyridine-4-carboxylic acid (2-morpholin-4-ylethyl)amide;
6-{2-[3-(3-methoxyphenyl)ureido]thiazol-5-yl}-1H-pyrazolo[3,4-b]pyridine-4-carboxylic acid (2-morpholin-4-ylethyl)amide;
6-{2-[3-(4-chlorophenyl)ureido]thiazol-5-yl}-1H-pyrazolo[3,4-b]pyridine-4-carboxylic acid (2-morpholin-4-ylethyl)amide;
6-{2-[3-(2,4-dimethoxyphenyl)ureido]thiazol-5-yl}-1H-pyrazolo[3,4-b]pyridine-4-carboxylic acid (2-morpholin-4-ylethyl)amide.

13. Product according to any one of Claims 1 to 11, **characterized in that** it is chosen from:
N-[4-(4-(2-morpholin-4-ylethyl)aminocarbonyl-1H-pyrazolo[3,4-b]pyridin-6-yl)phenyl]-3-chlorobenzenesulphonamide;
N-[4-(4-(piperazine-1-carbonyl)-1H-pyrazolo[3,4-b]pyridin-6-yl)phenyl]-2,3-dichlorobenzenesulphonamide;
N-[4-(4-methylaminocarbonyl-1H-pyrazolo[3,4-b]pyridin-6-yl)phenyl]-2-chloro-4-trifluoromethylbenzenesulphonamide;
N-[4-(4-methylaminocarbonyl-1H-pyrazolo[3,4-b]pyridin-6-yl)phenyl]-4-fluorobenzenesulphonamide;
N-[4-(3-amino-1H-pyrazolo[3,9-b]pyridin-6-yl)phenyl]-2,3-dichlorobenzenesulphonamide;
N-[4-(3-amino-4-methylaminocarbonyl-1H-pyrazolo[3,4-b]pyridin-6-yl)phenyl]-2,3-dichlorobenzenesulphonamide.

14. Product according to any one of the preceding claims, **characterized in that** it is in:
a) non-chiral form, or
b) racemic form, or
c) a form enriched in a stereoisomer, or
d) a form enriched in an enantiomer;
and **in that** it is optionally salified.

15. Medicament, **characterized in that** it comprises a product of formula (I) according to any one of Claims 1 to 14, or an addition salt of this compound with a pharmaceutically acceptable acid, or else a hydrate or a solvate of the product of formula (I).

16. Pharmaceutical composition comprising a product according to any one of the preceding claims, in combination with a pharmaceutically acceptable excipient.

17. Product according to any one of Claims 1 to 14, for use as an agent for inhibiting a reaction catalysed by one or more kinases.

18. Product for use according to Claim 17, **characterized in that** the kinase is chosen from FAK, KDR and Tie2.

19. Use of a product according to any one of Claims 1 to 14, for the manufacture of a medicament of use in the treatment of a pathological condition.

20. Use according to Claim 19, **characterized in that** the pathological condition is cancer.

21. Use according to Claim 19 or 20, **characterized in that** the pathological condition is chosen from rheumatoid arthritis, osteoarthritis and/or its associated pain, inflammatory diseases of the intestine, such as ulcerative colitis or Crohn's disease, eye pathologies such as age-related macular degeneration, diabetic retinopathies, chronic inflammation, psoriasis, and specific cancers such as Kaposi's sarcoma or infantile haemangioma.

22. Use, according to Claims 19 to 21, of a product according to any one of Claims 1 to 14, for the treatment or prevention of a pathological condition, **characterized in that** the product is administered alone or in combination with other active ingredients, in particular anticancer agents such as cytotoxic, cytostatic, anti-angiogenic or anti-metastatic products.
